# EUROPEAN PATENT APPLICATION

(11) **EP 4 509 523 A1**
(43) Date of publication of application: **19.02.2025**
(21) Application number: 23192200.6
(22) Date of filing: 18.08.2023
(51) Int. Cl.: C07K 14/705, C07K 14/725, A61K 39/00

(54) **REPAIRED KIR3DX1 AND ITS THERAPEUTIC USE**

(71) Applicant: Deutsches Primatenzentrum GmbH (DPZ), 37077 Göttingen (DE)
(72) Inventor: WALTER, Lutz, 37154 Northeim (DE); HASAN, Mohammad Zahidul, 37075 Göttingen (DE); PETERSEN, Beatrix, 37085 Göttingen (DE); ECKERMANN-FELKL, Ellen, 37115 Duderstadt (DE); WESTPHAL, Nico, 37079 Göttingen (DE)
(74) Representative: Moré, Solveig Helga

(57) **Abstract**

The present invention relates to killer cell immunoglobulin-like receptors (KIRs) and to immunotherapy against tumor and autoimmune diseases associated with an increased expression of the KIR3DL2 receptor. In particular, the present invention provides polypeptides and fusion proteins comprising an amino acid sequence having at least 85% sequence identity to SEQ ID NO: 1. This corresponds to a KIR3DX1 D2 domain wherein a deletion found in humans has been repaired. The invention discloses engineered KIR family receptors and CARs comprising the inventive polypeptides as antigen recognition domain. Further provided are nucleic acids encoding the polypeptides and receptors of the invention as well as cells expressing said receptors. The polypeptides, fusion proteins and cells of the invention as well as pharmaceutical compositions comprising the same may be used for the treatment of KIR3DL2-associated conditions such as rheumatic diseases including spondylarthritides or T cell leukemias including cutaneous T-cell lymphoma (CTCL), Sézary syndrome, mycosis fungoides, adult T-cell leukemia (ATL) or myelodysplastic syndrome. In a further aspect, the present invention also relates to methods for detecting KIR3DL2.

## Description

The present invention relates to killer cell immunoglobulin-like receptors (KIRs) and to immunotherapy against tumor and autoimmune diseases associated with an increased expression of the KIR3DL2 receptor. In particular, the present invention provides polypeptides and fusion proteins comprising an amino acid sequence having at least 85% sequence identity to SEQ ID NO: 1. This corresponds to a KIR3DX1 D2 domain wherein a deletion found in humans has been repaired. The invention discloses engineered KIR family receptors and CARs comprising the inventive polypeptides as antigen recognition domain. Further provided are nucleic acids encoding the polypeptides and receptors of the invention as well as cells expressing said receptors. The polypeptides, fusion proteins and cells of the invention as well as pharmaceutical compositions comprising the same may be used for the treatment of KIR3DL2-associated conditions such as rheumatic diseases including spondylarthritides or T cell leukemias including cutaneous T-cell lymphoma (CTCL), Sézary syndrome, mycosis fungoides, adult T-cell leukemia (ATL) or myelodysplastic syndrome. In a further aspect, the present invention also relates to methods for detecting KIR3DL2.

KIRs are germline-encoded type I transmembrane glycoprotein receptors expressed on the plasma membrane of natural killer (NK) cells and subsets of T lymphocytes. The members of this polymorphic receptor family encompass both inhibitory and stimulatory receptors that regulate the cytotoxic activity of these lymphocytes by interacting with major histocompatibility (MHC) class I molecules and other ligands expressed on target cells.

One representative of this family of receptors, KIR3DL2, is usually expressed by NK lymphocytes and, to a lesser percentage, also by CD4-positive helper T lymphocytes and CD8-positive cytotoxic T lymphocytes, especially after their activation. Human KIR3DL2 is distinguished from other KIRs by carrying two cysteine residues in the central stem region that lead to the formation of disulfide bridges and homodimerization of the KIR3DL2 receptor. KIR3DL2 acts an inhibitory KIR-family receptor. Its binding by a suitable ligand such as HLA, in particular HLA-A*03, HLA-A*11 and HLA-B*27, mediates an inhibition of IFN-y production and various cytotoxic functions and controls NK cell development and maturation. For binding to HLA-A*03 and HLA-A*11, this process appears to be dependent on the presence of a specific EBV peptide. While the exact significance of this peptide dependence remains still unclear, it is suggested that it is of regulatory function.

Binding of a ligand on KIR3DL2 expressed on T lymphocytes acts in concert with, e.g., other T cell receptor (TCR) mediated responses. For example, on activated T cells after ligand binding (e.g., HLA-B*27), KIR3DL2 can have an anti-apoptotic effect and trigger the production of IL-17. IL-17 has an antitumor effect as a pro-inflammatory cytokine. On the other hand, IL-17 has also been shown to increase carcinogenesis, metastasis and resistance to chemotherapy in various tumor types.

In recent years, several additional KIR3DL2 ligands have been identified. For instance, KIR3DL2 was found to bind to CpG oligodeoxynucleotides (ODNs) via its D0 extracellular domain. KIR3DL2 is subsequently endocytosed and shuttles the CpG ODNs to the endosomal compartment where the CpG cargo is loaded on Toll-like receptor 9 (TLR9) (Sivori et al., 2010a). This process leads to expression of IFN-y and to elevated cytotoxicity in NK cells, thereby triggering the initiation of an inflammatory response in KIR3DL2-positive cells. As CpG-ODNs include DNA containing an unmethylated cytosine followed by a guanine, they mimic bacterial DNA, i.e., they are a type of pathogen-associated molecular patterns (PAMPs). Accordingly, KIR3DL2 is suspected to contribute to the immune response to microbial infections.

All of the above-mentioned features suggest that KIR3DL2 plays an important role in various diseases. Indeed, the interaction between KIR3DL2 expressed on CD4⁺ T helper (Th) cells and the free heavy chain form of HLA-B*27, which appears to occur independent of the presence of additional peptides such as EBV peptides, leads to the differentiation of the T cells into pro-inflammatory Th17 cells that produce IL-17, which is considered to drive pathogenesis and progression of a group of rheumatic diseases known as spondylarthritides (Ridley et al., 2016). This mechanism could serve as an explanation for why the HLA-B allele HLA-B*27 is strongly associated with the occurrence of rheumatic diseases, e.g., ankylosing spondylitis.

Moreover, KIR3DL2 was found to be expressed on the surface of malignant T cells in various T-cell leukemias including cutaneous T-cell lymphoma (CTCL), Sézary syndrome, mycosis fungoides, or adult T-cell leukemia (ATL) (Bagot et al., 2001; Schmitt et al., 2017, Marie-Cardine et al., 2014; Hurabielle et al., 2018). Increased expression of KIR3DL2 has been further described in malignant disorders of hematopoiesis such as myelodysplastic syndrome.

The association with the aforementioned diseases renders KIR3DL2 a biomarker for their diagnosis and monitoring as well as an interesting therapeutic target for their treatment.

To date, however, only one therapeutic monoclonal antibody targeting KIR3DL2 has been developed. This antibody, known as lacutamab or IPH4102 has been tested in a phase 1 clinical trial for the treatment of CTCL (NCT02593045). In another study, NCT03322618, the pathophysiological role of KIR3DL2 in axial spondyloarthritis and its relationship to Th17 immunity in HLA-B*27 positive and negative patients was tested. The study further monitored the effect on the pro-inflammatory immune response of IPH4102 in these patients. Additional phase I and phase II clinical trials for treating T cell lymphomas using lacutamab are currently planned or ongoing.

However, it is a well-known problem that tumors can escape the binding of antibodies by mutation. Another therapeutic agent using a different binding site may therefore help to continue fighting the tumor cells despite the emergence of escape mutations against the first therapeutic agent. Sequential treatments with different therapeutics are therefore preferred in tumor therapies. For KIR3DL2-based immunotherapies, these successive treatments do however not yet exist. In the case of inflammatory diseases such as rheumatism or sepsis, a long-term therapy with cortisone is often successful, but it is frequently associated with severe side effects. It would therefore be desirable to make available further agents that have a more targeted and, above all, a modulating effect on the excessive immune reactions in rheumatic diseases as well as in sepsis or so-called superinfections.

Collectively, there is thus a great need to identify novel highly effective drugs that can target and specifically bind KIR3DL2 and may thus be used for the treatment of KIR3DL2-associated T-cell lymphomas and/or rheumatoid diseases.

This problem is solved by the present invention, in particular by the subject-matter of the claims.

### Polypeptides of the invention

In a first aspect, the present invention provides a polypeptide comprising an amino acid sequence having at least 85% sequence identity to SEQ ID NO: 1, wherein the polypeptide does not comprise the amino acid sequence of the chimpanzee, gorilla, orangutan, bonobo or siamang KIR3DX1 D2 domains of SEQ ID NO: 15, 16, 17, 36 or 60 respectively.

SEQ ID NO: 1 corresponds to a repaired version of the D2 extracellular domain of human KIR3DX1. The killer cell immunoglobulin-like receptor (*KIR)* genes in simian primates (including humans, apes, Old World monkeys, New World monkeys) and bovids (cattle, goats, antelopes) were shown to be diversified and to belong to two ancient *KIR* lineages: while simian primates have expanded and diversified the *KIR3DL* lineage and kept *KIR3DX1* as a conserved single-copy gene, the opposite was found in bovid species where the *KIR3DX* lineage is expanded (Guethlein et al., 2007; Guethlein et al., 2015). The divergence of these two *KIR* lineages was estimated to have occurred about 135 million years ago, thus predating the diversification of placental mammals. *KIR3DX1* was first described in humans under its original name *KIR3DL0* and was found about 180 kb centromeric to the *KIR3DL* cluster on human chromosome 19q13.4 (Sambrook et al., 2006). Notably, the initial description of human *KIR3DX1* reported a 7 bp deletion in exon 5 that encodes the extracellular immunoglobulin-like D2 domain. Sequencing of 86 unrelated individuals revealed only the deleted form and no undeleted *KIR3DX1* allele (Sambrook et al., 2006), suggesting fixation of the deletion in the human genome. In contrast, a corresponding deletion was not found in chimpanzee, gorilla, rhesus macaque and common marmoset where *KIR3DX1* encodes a fully functional inhibitory KIR-family receptor (Sambrook et al., 2006). The deletion in human *KIR3DX1* leads to a shift in the open reading frame and premature termination. Accordingly, the putative amino acid sequence encoded by the human wild type *KIR3DX1* gene comprises the two extracellular immunoglobin domains D0 and D1 as well as a truncated domain D2, but completely lacks the typical stem, transmembrane and cytoplasmic regions of KIR family proteins. It was therefore speculated that the human KIR3DX1 protein is not a typical cell surface receptor and may be secreted. Nevertheless, prior to the present invention, the exact function of KIR3DX1 in primates and the reason for the deletion in human KIR3DX1 were completely unknown. This formed the starting point of the work that led to the present invention.

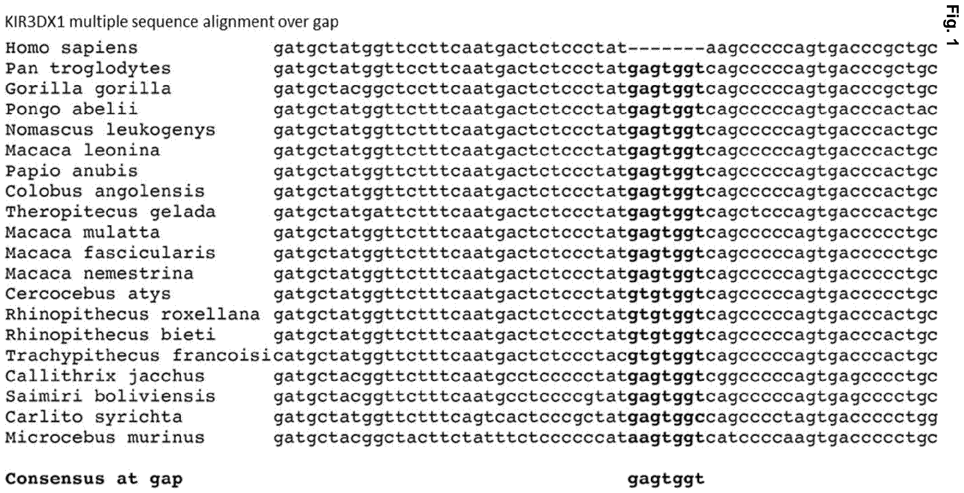

As described in detail in the example section below, the present inventors generated a repaired version of human KIR3DX1 by first identifying the seven deleted nucleic acids in exon 5 of the human KIR3DX1 gene. To do this, they compared currently available primate genome sequences and surprisingly discovered that the deleted sequence is highly conserved in non-human primates (Fig. 1).

Re-introduction of this 7-nucleotide consensus sequences into the human wild type *KIR3DX1* gene resulted in the expression of a full-length KIR3DX1 receptor comprising the three extracellular domains D0, D1 and D2, as well as a stem region, a transmembrane region and a cytoplasmic tail. In the context of the invention, the full-length human KIR3DX1 receptor expressed upon re-introduction of the 7-nucleotide deletion into the wild type *KIR3DX1* gene is referred to as KIR3DX1-repaired or simply as KIR3DX1rep. As detailed in the examples below, the inventors surprisingly found that KIR3DX1rep can specifically bind to KIR3DL2. Fine-mapping of the interaction site and mutation studies of KIR3DL2 revealed this specific binding to depend on the D2 domain of repaired KIR3DX1, i.e. the particular domain that is affected by the 7bp deletion in wild type *KIR3DX1.* Moreover, the binding interaction of repaired KIR3DX1 to KIR3DL2 involves a glycine residue at position 56 of the D0 domain of KIR3DL2 and further depends on the ability of KIR3DL2 to undergo homodimerization.

Based on the unexpected finding that the D2 domain of repaired human KIR3DX1 (KIR3DX1rep) can bind to KIR3DL2, the inventors considered its therapeutically use as a competitive, i.e., an antagonistic inhibitor of KIR3DL2.

In one embodiment, the polypeptide of the present invention corresponds to the D2 domain encoded by the repaired human KIR3DX1 gene, as described herein. The polypeptide thus preferably comprises an amino acid sequence of SEQ ID NO: 1. In some embodiments, the polypeptide of the invention may also consist of an amino acid sequence of SEQ ID NO: 1.

However, the amino acid sequence of the polypeptide may also comprise mutations, such as 1 to 15, e.g., 1, 2, 3, 4, 5, 6, 7, 8, 9, 10, 11, 12, 13, 14, or 15 amino acid substitutions as compared to SEQ ID NO: 1. Accordingly, the polypeptide of the invention may therefore comprise or consist of an amino acid sequence having at least 85%, at least 86%, at least 87%, at least 88%, at least 89%, at least 90%, at least 91%, at least 92%, at least 93%, at least 94%, at least 95%, at least 96%, at least 97%, at least 98% or at least 99% sequence identity to SEQ ID NO: 1, wherein, however, the amino acid sequence of the polypeptide according to the invention does not correspond to the amino acid sequence of a non-human primate KIR3DX1 D2 domain or a bovine KIR3DX1 D2 domain. In particular, the polypeptide of the invention comprising or consisting of an amino acid having at least 85% sequence identity to SEQ ID NO: 1 does not comprise the amino acid sequence of chimpanzee, gorilla, orangutan, bonobo or siamang KIR3DX1 D2 corresponding to SEQ ID NO: 15, 16, 17, 36 and 60, respectively.

The polypeptide comprising or consisting of an amino acid sequence having at least 85% sequence identity to SEQ ID NO: 1 as described herein is capable of binding to human KIR3DL2. The ability of the polypeptide to interact and bind to KIR3DL2 can be easily verified using standard in vitro protein-protein interaction assays such as e.g., co-immunoprecipitation followed by western blotting. Briefly, such an assay may comprise steps of contacting a polypeptide of the invention with purified KIR3DL2 or a cell expressing KIR3DL2 on its surface. The KIR3DL2 may then be isolated using a specific antibody and binding of the polypeptide of the invention can be detected by western blotting. Typically, the different proteins that are to be tested for their binding are modified to carry distinct protein tags such as HA-tags, V5-tags or a FLAG-tags so that conventional antibodies against these tags can be used for isolation and detection. The polypeptide of the invention may also be fused to the Fc portion of IgG, as described in detail below to facilitate its purification and detection. Alternative methods for studying protein-protein interactions are well-known to the skilled person and include, e.g., pull-down assays.

The inventors could demonstrate that the part of the KIR3DX1 D2 domain which is normally deleted in the truncated wild type human protein is involved in mediating the interaction with KIR3DL2 (see the example section below). This part of D2 consists of a stretch of 13 amino acids having the consensus sequence EWSX₁X₂SDPLX₃LYX₄ (SEQ ID NO: 2), wherein X₁, X₃ and X₄ can be any naturally occurring amino acid, and X₂ is S or P, preferably P. X₁ preferably is A. X₃ optionally is Q or H, preferably Q. X₄ optionally is T, A or V, preferably T. Accordingly, the polypeptide according to the invention preferably comprises or consists of an amino acid sequence having at least 85% sequence identity to SEQ ID NO: 1 that does not comprise the amino acid sequence of chimpanzee, gorilla, orangutan, bonobo or siamang KIR3DX1 D2, while comprising amino acid sequence SEQ ID NO: 2.

More preferably, the polypeptide according to the invention comprises or consists of an amino acid sequence having at least 85% sequence identity to SEQ ID NO: 1 while comprising the amino acid sequence EWSAPSDPLQLYT (SEQ ID NO: 3, reconstructed human D2). In another embodiment, the polypeptide according to the invention comprises or consists of an amino acid sequence having at least 85% sequence identity to SEQ ID NO: 1 while comprising the amino acid sequence EWSAPSDPLQLYA (SEQ ID NO: 4). SEQ ID NO: 4 is present in the chimpanzee, gorilla and bonobo D2 domain. In yet another embodiment, the polypeptide according to the invention comprises or consists of an amino acid sequence having at least 85% sequence identity to SEQ ID NO: 1 while comprising the amino acid sequence EWSAPSDPLHLYV (SEQ ID NO: 39). SEQ ID NO: 39 is present in the orangutan and siamang D2 domain.

In any case, however, the polypeptide of the invention preferably comprises or consist of an amino acid sequence having at least 85% sequence identity to SEQ ID NO: 1 while comprising at least the consensus sequence WSX₁X₂S, wherein X₁ is any natural amino acid, preferably, A, and X₂ is S or P, preferably P. Without being bound by theory, this shorter consensus sequence is suspected to form a beta bulge that may be required for mediating the interaction between the D2rep domain and the D0 domain of KIR3DL2.

Preferably, the polypeptide of the invention further comprises a first cysteine residue at amino acid position 26 and a second cysteine residue at amino acid position 77, wherein these "positions" refer to the respective location within the amino acid sequence of SEQ ID NO: 1 and are thus determined in relation to the D2 domain portion of the inventive polypeptide only.

Corresponding cysteine residues are frequently found in members of the Ig superfamily, where disulfide bridges contribute to the typical immunoglobulin-like fold.

In the context of the invention, a polypeptide capable of binding to human KIR3DL2 consisting of an amino acid sequence having at least 85% sequence identity to SEQ ID NO: 1 and preferably comprising at least the consensus sequence WSX₁X₂S as described above, and which does not comprise the amino acid sequence of chimpanzee, gorilla, orangutan, bonobo or siamang KIR3DX1 D2, is referred to as D2rep. Most preferably, D2rep comprise the beta bulge motif WSAPS as present, e.g., in chimpanzee, bonobo, gorilla, orangutan, siamang as well as the reconstructed human sequence. In a preferred embodiment, D2rep also comprises SEQ ID NO: 2, e.g., SEQ ID NO: 3, SEQ ID NO: 4 or SEQ ID NO: 39, preferably, SEQ ID NO: 3.

A polypeptide comprising D2rep was found to be sufficient to bind KIR3DL2. Accordingly, as described in more detail below, a polypeptide comprising or consisting of D2rep may be used as an inhibitor to interfere with the interaction of KIR3DL2 with its ligands. For instance, by binding to KIR3DL2 on CD4⁺ Th cells, the polypeptide according to the invention can interfere with the interaction of KIR3DL2 and HLA-B*27, and thus prevent the differentiation of the KIR3DL2-expressing CD4⁺ T cells into pro-inflammatory Th17 cells. The polypeptide according to the invention may also interfere with the interaction of KIR3DL2 and bacterial DNA, thereby preventing the loading of the bacterial DNA on TLR9 and consequently reducing the TLR9-mediated inflammatory response mediated by KIR3DL2-positive cells. Preventing or at least reducing CpG-mediated inflammation may be particularly useful in bacterial infection-related clinical conditions characterized by exaggerated immune responses, such as sepsis or bacterial and viral dual infections (so-called superinfections). The peptide according to the invention may also be used for scientific or diagnostic purposes, e.g., for labelling cells that express KIR3DL2 on their surface or in ELISA-based diagnostic assays for the detection of soluble KIR3DL2 in patient samples.

In addition to comprising D2rep, the polypeptide according to the invention may further comprise a D0 domain of KIR3DX1 consisting of an amino acid sequence having at least 70%, e.g., at least 75%, at least 80%, at least 85%, at least 90% or at least 95% sequence identity to SEQ ID NO: 5. The polypeptide may also comprise a KIR3DX1 D0 domain consisting of an amino acid sequence having 100% sequence identity to SEQ ID NO: 5.

Alternatively or in addition, the polypeptide according to the invention comprising D2rep may further comprise a D1 domain of KIR3DX1 consisting of an amino acid sequence having at least 70%, e.g., at least 75%, at least 80%, at least 85%, at least 90% or at least 95% sequence identity to SEQ ID NO: 6. The polypeptide may also comprise a KIR3DX1 D1 domain consisting of an amino acid sequence having 100% sequence identity to SEQ ID NO: 6.

Preferably, however, the polypeptide according to the invention comprises D0, D1 and D2rep domains, or the entire extracellular region of KIR3DX1rep. It may thus comprise D2rep and both the KIR3DX1 D0 domain consisting of an amino acid sequence having at least 70% of SEQ ID NO: 5 and the KIR3DX1 D1 domain consisting of an amino acid sequence having at least 70% of SEQ ID NO: 6. In such an embodiment, the polypeptide of the invention thus comprises all three extracellular immunoglobulin domains D0, D1 and D2 of KIR3DX1 and may therefore comprise an amino acid sequence of SEQ ID NO: 7. The polypeptide of the invention may also consist of SEQ ID NO: 7. Optionally, it further comprises the stem region of KIR3DX1, e.g., an ape KIR3DX1, such as chimpanzee, bonobo, orangutan, siamang or gorilla.

Shorter polypeptides, e.g., D2rep only, may be easier and cheaper to produce than longer proteins. However, longer polypeptide versions may have a better functionality in some aspects, e.g., in fusion proteins. They may also result in better detection of KIR3DL2, e.g., the presence of the D0 and/or D1 domain in addition to D2rep may increase the binding strength of the polypeptide according to the invention to KIR3DL2. As described in the examples below, the inventors found that commercially available anti-KIR3DL2 antibodies detect KIR3DL2 less effectively in the presence of KIR3DX1 D0-D1-D2rep-Fc proteins or upon simultaneous expression of KIR3DL2 and full-length KIR3DX1rep in cells.

### Fusion proteins

Regardless of whether the polypeptide of the invention comprises only D2rep or additionally also the D0 and/or the D1 domain of KIR3DX1 as described herein, in some embodiments, it may be provided in the form of a fusion protein.

Thus, in one embodiment, the polypeptide of the invention is fused to the Fc (fragment crystallizable) region of an immunoglobulin.

The Fc region constitutes the tail region of an immunoglobulin or antibody. In IgG, IgA and IgD antibody isotypes, the Fc region is composed of two identical protein fragments, derived from the second and third constant domains of the antibody's two heavy chains. When an antibody binds to a target cell, its Fc region interacts with cell surface receptors, Fc receptors, located on the surface of particular immune effector cells to activate the immune system. For instance, effector cells such as NK cells can recognize the Fc region of cell-bound IgG via their Fcy receptor CD16, which results in the NK cell's degranulation and subsequent release of cytotoxic factors to kill the targeted cell in a process known as antibody-dependent cellular toxicity (ADCC). CD16 is also expressed on subsets of macrophages and monocytes, which can thus also initiate ADCC and cytokine release upon FC recognition.

Thus, a polypeptide of the invention fused to an antibody Fc region may be used to specifically induce the killing of KIR3DL2-expressing cells via ADCC.

Accordingly, in a preferred embodiment, the polypeptide of the invention is a fusion protein that comprises at least D2rep fused to an immunoglobulin Fc region capable of inducing ADCC when being recognized by a natural killer (NK) cell or macrophage/monocyte. Preferably, the polypeptide of the invention comprises an IgG-Fc region, most preferably the Fc region of IgG subclass IgG1 as this subclass exhibits the highest Fcy receptor-binding affinity and thus is the most potent inducer of ADCC. Therefore, in a particular preferred embodiment, the polypeptide of the invention is a fusion protein that comprises at least D2rep and is fused to an IgG1-Fc region, wherein the IgG1 Fc region may have an amino acid sequence of SEQ ID NO: 8 or at least 90% sequence identity thereto. The polypeptide of the invention may however also be fused to another immunoglobulin Fc region capable of inducing ADCC, e.g. an Fc region of IgG3.

However, in some embodiments, it may be desirable that the polypeptide of the invention is a fusion protein comprising an Fc domain of an antibody that does not or only weakly mediates the induction of ADCC. For instance, IgG subclass IgG4 has weak affinities for most Fc receptors and therefore is a poor inducer of Fc-mediated effector functions (Yu et al., 2020). Thus, the polypeptide of the invention may, in some embodiments, also comprise the Fc region of IgG4 having an amino acid sequence of SEQ ID NO: 9 or having at least 90% sequence identity thereto. Such a fusion-protein could be used for example for research and diagnostic purposes, e.g., for labeling cells expressing KIR3DL2 on their surface or in ELISA-based diagnostic assays. However, a polypeptide according to the invention comprising an Fc region that does not induce ADCC may also be used as an antagonistic inhibitor that is able to interfere with the interaction of KIR3DL2 with its ligands, as described herein.

The human D2rep protein as described herein exhibits high sequence similarity to the wild-type D2 domains of chimpanzee, gorilla, orangutan, bonobo and siamang KIR3DX1. Especially the reconstructed 13-amino acid region of D2rep required for interaction with human KIR3DL2, which is normally absent in the wild-type human D2 domain due to the deletion in exon 5 of the human *KIR3DX1* gene and the ensuing frame-shift shares high sequence similarity with the corresponding region in chimpanzee, gorilla, orangutan, bonobo and siamang D2. In consequence, the KIR3DX1 D2 domain of these five primate species is able to bind to human KIR3DL2. In contrast, the KIR3DX1 D2 domain of less related species such as rhesus monkey does not bind to human KIR3DL.

Thus, also disclosed is a fusion protein comprising at least the D2 domain of chimpanzee, gorilla, orangutan, bonobo or siamang KIR3DX1 fused to an Fc region of an immunoglobin, preferably any Fc region capable of inducing ADCC.

For instance, the fusion protein may comprise the D2 domain of chimpanzee KIR3DX1 corresponding to SEQ ID NO: 15 fused to an IgG1-Fc region, e.g., having an amino acid sequence of SEQ ID NO: 8. Said fusion protein is able to induce ADCC of a cell expressing human KIR3DL2. The fusion protein may in addition comprise the D0 and/or D1 domain of human or chimpanzee KIR3DX1, e.g. it may be a polypeptide comprising the D0, D1 and D2 domain of chimpanzee KIR3DX1 according to SEQ ID NO: 18 fused to an IgG1-Fc region, e.g., having an amino acid sequence of SEQ ID NO: 8. The fusion protein comprising at least the D2 domain of chimpanzee KIR3DX1 may instead also comprise an Fc domain that does not significantly induce ADCC such as, e.g., an IgG4 Fc domain.

Alternatively, the fusion protein may comprise the D2 domain of gorilla KIR3DX1 corresponding to SEQ ID NO: 16 fused to an IgG1-Fc region, e.g., having an amino acid sequence of SEQ ID NO: 8. Said fusion protein is also able to induce ADCC of a cell expressing human KIR3DL2. The fusion protein may in addition comprise the D0 and/or D1 domain of human or gorilla KIR3DX1, e.g. it may be a polypeptide comprising the D0, D1 and D2 domain of gorilla KIR3DX1 according to SEQ ID NO: 19 fused to an IgG1-Fc region, e.g., having an amino acid sequence of SEQ ID NO: 8. The fusion protein comprising at least the D2 domain of gorilla KIR3DX1 may instead also comprise an Fc domain that does not significantly induce ADCC such as, e.g., an IgG4 Fc domain.

In another embodiment, the fusion protein may comprise the D2 domain of orangutan KIR3DX1 corresponding to SEQ ID NO: 17 fused to an IgG1-Fc region, e.g., having an amino acid sequence of SEQ ID NO: 8. Said fusion protein is also able to induce ADCC of a cell expressing human KIR3DL2. The fusion protein may also comprise the D0 and/or D1 domain of human or orangutan KIR3DX1, e.g. it may be a polypeptide comprising the D0, D1 and D2 domain of orangutan KIR3DX1 according to SEQ ID NO: 20 fused to an IgG1-Fc region, e.g., having an amino acid sequence of SEQ ID NO: 8. The fusion protein comprising at least the D2 domain of orangutan KIR3DX1 may instead also comprise an Fc domain that does not significantly induce ADCC such as, e.g., an IgG4 Fc domain.

In yet another embodiment, the fusion protein may comprise the D2 domain of bonobo KIR3DX1 corresponding to SEQ ID NO: 36 fused to an IgG1-Fc region, e.g., having an amino acid sequence of SEQ ID NO: 8. Said fusion protein is able to induce ADCC of a cell expressing human KIR3DL2. The fusion protein may in addition comprise the D0 and/or D1 domain of human or bonobo KIR3DX1, e.g. it may be a polypeptide comprising the D0, D1 and D2 domain of bonobo KIR3DX1 according to SEQ ID NO: 37 fused to an IgG1-Fc region, e.g., having an amino acid sequence of SEQ ID NO: 8. The fusion protein comprising at least the D2 domain of bonobo KIR3DX1 may instead also comprise an Fc domain that does not significantly induce ADCC such as, e.g., an IgG4 Fc domain.

In another embodiment, the fusion protein may comprise the D2 domain of siamang KIR3DX1 corresponding to SEQ ID NO: 60 fused to an IgG1-Fc region, e.g., having an amino acid sequence of SEQ ID NO: 8. Said fusion protein is able to induce ADCC of a cell expressing human KIR3DL2. The fusion protein may in addition comprise the D0 and/or D1 domain of human or siamang KIR3DX1, e.g. it may be a polypeptide comprising the D0, D1 and D2 domain of siamang KIR3DX1 according to SEQ ID NO: 61 fused to an IgG1-Fc region, e.g., having an amino acid sequence of SEQ ID NO: 8. The fusion protein comprising at least the D2 domain of siamang KIR3DX1 may instead also comprise an Fc domain that does not significantly induce ADCC such as, e.g., an IgG4 Fc domain.

In the context of the invention, the term fusion protein also extends to so-called multispecific engagers comprising a polypeptide of the invention, e.g., at least D2rep, or a polypeptide comprising at least the D2 domain of any of chimpanzee, gorilla, orangutan, bonobo or siamang KIR3DX1 as one of their antigen-binding domains. The term multispecific engager relates to an artificial protein that typically is constructed from the variable regions of at least two antigen-binding moieties, e.g., ligands and/or antibodies of different specificity. The variable region of an antibody is formed by a heavy (VH) and light (VL) chain and forms the antibody's antigen binding site. Accordingly, a multispecific engager can simultaneously bind to at least two different types of antigen or two different epitopes on the same antigen. These constructs can thus be used, e.g., to bring different cells into direct contact.

The present invention thus further provides a fusion protein in which a polypeptide according to the invention is linked to at least one additional antigen-binding moiety to form a multispecific engager. The additional antigen-binding moiety may be, e.g., a variable region of an antibody or a ligand of a receptor of interest. For instance, in one embodiment, the fusion protein comprises D2rep as a first antigen binding moiety linked to the variable region of an antibody capable of binding and engaging CD16 as a second antigen-binding moiety. Such a bispecific engager (also referred to as bispecific killer engager or BiKe) can be used to bring a CD16-expressing NK cell or monocyte/macrophage into contact with a KIR3DL2-expressing tumor cell to trigger killing of the tumor cell via ADCC. In another exemplary embodiment, the polypeptide of the invention, e.g., D2rep, may instead be linked to an antigen-binding moiety capable of engaging with the CD3 receptor expressed, e.g., on the surface of cytotoxic T cells. Such a fusion protein constitutes a so-called bispecific T-cell engager (BiTE).

The polypeptide of the invention may also be linked to more than one additional antigen-binding moiety, e.g., it may be linked to two, three, four, five or more antigen-binding moieties of different specificity. For instance, the fusion protein may be a trispecific killer engager (TriKe) comprising a polypeptide of the invention, e.g., D2rep, as a first antigen-binding moiety linked to two further antigen-binding moieties capable of engaging the activating receptors NKp46 and CD16 expressed by NK cells. Optionally, such a fusion protein may additionally comprise even a fourth antigen-binding moiety (tetra-specific engager) capable of binding, e.g., another tumor-associated antigen besides KIR3DL2 or of engaging with a receptor of another immune effector cell, such as the CD3 receptor or IL-2 receptor expressed by cytotoxic T cells or CD4+ helper T cells.

Preferably, the polypeptide of the invention and the at least one additional antigen-binding moiety are linked via a suitable polypeptide linker. The linker allows separation of the polypeptide of the invention and the antigen-binding moieties and provides flexibility to bind the multiple epitopes on the different targeted cells. Suitable flexible linkers are known from the state of the art, e.g., from Felices et al., 2016.

In alternative embodiments, the multispecific engager as described herein may also comprise the D2 domain and, optionally, the D0 and/or D1 domain of chimpanzee, gorilla, orangutan, bonobo or siamang KIR3DX1 instead of a polypeptide of the invention.

### Receptor constructs

In further embodiments, the polypeptide according to the invention may also be a functional killer cell immunoglobulin-like receptor (KIR). It thus typically does not comprise an Fc domain or additional antigen-binding moieties, but instead the stem region, the transmembrane region and the cytoplasmic region of a KIR family protein. However, in some embodiments, the KIR according to the invention may additionally also be fused to an Fc region of an antibody, e.g., an IgG1 or an IgG4 Fc region.

In a preferred embodiment, the polypeptide of the invention thus comprises the D2rep domain and, preferably, also the KIR3DX1 D0 and/or D1 domains as described herein, as well as a KIR3DX1 stem region comprising an amino acid sequence having at least 70%, e.g., at least 80%, at least 90%, or 100% sequence identity to SEQ ID NO: 10, a KIR3DX1 transmembrane region comprising an amino acid sequence having at least 70%, e.g., at least 80%, at least 90%, or 100% sequence identity to SEQ ID NO: 11 and a KIR3DX1 cytoplasmic region comprising an amino acid sequence having at least 70%, e.g., at least 75%, at least 80%, at least 85%, at least 90%, at least 95% or 100% sequence identity to SEQ ID NO: 12. Preferably, the D0, D1 and/or D2 domains are human to prevent or at least to minimize an immune response, but the stem region, the transmembrane region and/or the cytoplasmic region may be the reconstructed human sequence or an ape sequence, such as chimpanzee, bonobo, orangutan, siamang or gorilla sequence. Optionally, such a polypeptide comprises all three extracellular domains D0, D1 and D2rep as described herein so that the full-length human KIR3DX1rep receptor is obtained. Such a construct may have an amino acid sequence corresponding to SEQ ID NO: 13.

In some embodiments, the KIR3DX1rep receptor may also be a chimeric KIR receptor. For instance, while the receptor comprises at least the D2rep domain of human KIR3DX1 as disclosed herein, any of the D0 domain, the D1 domain, the stem region, the transmembrane region and/or the cytoplasmic region, in particular, the cytoplasmic region of the receptor may be replaced by corresponding domains/regions of one or more other members of the human KIR-family of receptors, such as, e.g., KIR2DL1, KIR2DL2, KIR2DL3, KIR2DL4, KIR2DL5A, KIR2DL5B, KIR2DS1, KIR2DS2, KIR2DS3, KIR2DS5, KIR2DP1, KIR3DP1, KIR3DL1, KIR3DS1, KIR3DL2, or KIR3DL3. For instance, the polypeptide according to invention may comprise the extracellular KIR3DX1 D2rep domain and the D0 domain, the D1 domain, the stem region, the transmembrane region and/or the cytoplasmic region of KIR3DL2. The receptor may also comprise a complete extracellular domain of human KIR3DX1rep comprising KIR3DX1 D0, D1 and D2rep, and optionally the stem region, with the transmembrane region and/or the cytoplasmic region (and, if not yet present, the stem region) from one or more other human KIR family proteins. In some embodiments, one, two, three, or four of D0, D1, the stem region, the transmembrane region, and the cytoplasmic region are derived from a KIR family member other than KIR3DX1, while the remainder of the receptor is derived from the repaired human KIR3DX1rep. Given that KIR3DX1 is considered to be an inhibitory KIR family receptor due to the presence of the immunoreceptor tyrosine-based inhibitory motif (ITIM) in its cytoplasmic region, in some embodiments, at least its transmembrane and cytoplasmic regions may be replaced by the corresponding regions of an activating KIR family member such as, e.g., KIR2DS1, KIR2DL4, and KIR3DS1. These activating receptors do not comprise an ITIM (with the exception of KIR2L4) and contain a positively charged lysine or arginine residue in their transmembrane domain that helps to bind adapter proteins such as DAP12 or FCER1G, containing a negatively charged residue as well as immunoreceptor tyrosine-based activation motifs (ITAM). The resulting chimeric KIR would thus be able to recognize KIR3DL2 via its extracellular domain comprising D2rep but would in turn activate rather than inhibit the cytotoxic activity of a cell expressing the receptor. An exemplary receptor comprising an extracellular domain of human KIR3DX1rep and the transmembrane as well as cytoplasmic region of the activating human KIR family receptor KIR3DS1 has an amino acid sequence of SEQ ID NO: 31.

In the chimeric KIR receptor, at least the D2rep domain of human KIR3DX1 may also be combined with any or all of the D0 domain, D1 domain, stem region, transmembrane region, and cytoplasmic region derived from a non-human KIR family protein, i.e., a non-human primate KIR family protein or a bovid KIR family protein.

The present invention also provides a chimeric antigen receptor (CAR) comprising a polypeptide of the invention as an antigen recognition domain. In other words, the polypeptide according to the invention comprising D2rep may form the antigen recognition domain of a CAR construct.

The term chimeric antigen receptor or CAR refers to an artificial chimeric protein comprising an extracellular antigen-binding/recognition domain that in other cases typically is a single chain construct derived from an antibody, a transmembrane domain, an intracellular signalling domain and additional costimulatory domains from receptors such as CD28, OX40, and CD137. Binding of an antigen to the antigen-binding domain causes the clustering of CARs and the subsequent transmission of an activation signal. CARs are typically used to modify the antigen specificity of T cells or NK cells to target cancer cells in immunotherapy.

Preferably, the present invention provides a CAR comprising at least the KIR3DX1 D2rep domain, optionally, the KIR3DX1 D2rep domain as well as the KIR3XD1 D0 and D1 domains as described herein as an antigen-binding domain. Accordingly, in a preferred embodiment, the CAR comprises the entire extracellular portion of KIR3DX1rep.

In one embodiment, the CAR comprises the KIR3DX1 D2rep domain without the KIR3XD1 D0 and D1 domains.

The CAR may optionally comprise a hinge or spacer domain located between the antigen recognition domain and the transmembrane domain to provide more flexibility and accessibility for the antigen-binding domain. The hinge region may, e.g., be an Fc region of an antibody such as IgG or an immunoglobulin-like domain or hinge domain derived from the extracellular portion of, e.g., CD8 or CD28. It may also be a stem region of a KIR, e.g., of KIR3DX1.

The transmembrane domain of the CAR provided herein anchors the CAR to the plasma membrane of a cell and ensures the stability of the receptor as a whole. Suitable transmembrane domains that are conventionally used in CAR constructs may be derived from, e.g., CD28, ICOS, CD4, CD8 or CD3ζ.

The CAR preferably comprises an intracellular signalling domain derived from the cytoplasmic domain of CD3ζ. In some embodiments, the CAR may be a first-generation CAR comprising only this CD3ζ intracellular signalling domain. However, since T-cells typically require co-stimulation in addition to CD3 signalling, the CAR preferably is a second or third generation CAR, i.e., the intracellular domain preferably comprises at least one additional co-stimulatory domain. The at least one co-stimulatory domain may be derived from, e.g., CD28, 4-1 BB (also known as CD137), or OX40.

The skilled person is readily able to select suitable transmembrane, intracellular signalling, co-stimulatory and, optionally, hinge domains for the CAR.

In a preferred embodiment, the CAR comprises the D0, D1 and D2 domains of KIR3DX1rep as an antigen recognition domain, the stem of KI R3DX 1 rep as a hinge region, a transmembrane and cytoplasmic region of CD28 and a CD3ζ intracellular signaling domain. The preferred CAR of the invention thus may have an amino acid sequence corresponding to SEQ ID NO: 14.

The CAR according to the invention may also comprise an antigen recognition domain comprising at least the wild type D2 domain of chimpanzee, gorilla, orangutan, bonobo or siamang KIR3DX1. Accordingly, the herein disclosed CAR may also comprise an antigen-recognition domain comprising an amino acid sequence of SEQ ID NO: 15, 16, 17, 36 or 60. Most preferably, the CAR comprises the entire extracellular domain of chimpanzee, gorilla, orangutan, bonobo or siamang KIR3DX1 as an antigen-recognition domain, i.e. the antigen-recognition domain of the CAR comprises the D0, D1 and D2 domain of wild type chimpanzee, gorilla, orangutan, bonobo or siamang KIR3DX1 and thus any of the amino acid sequences of SEQ ID NO: 18, 19, 20, 37 or 61. Optionally, it also comprises the stem region of one of these species, preferably, from the same species.

### Nucleic acids

The present invention further provides a nucleic acid encoding any of the polypeptides, fusion proteins and receptors disclosed herein. The nucleic acid can either be DNA or RNA. Preferably, it is DNA.

Importantly, the nucleic acid encoding the polypeptide of the invention includes an additional number of nucleotides that repairs the frameshift that occurred in the human gene compared to the other primate species. Accordingly, compared to the human wildtype sequence, it may include additional seven, four or one nucleotides, preferably, at the position where the sequence was deleted, as shown in Fig. 1 and discussed below.

The nucleic acid preferably comprises the seven nucleotides that are deleted in the human wild type *KIR3DX1* gene but are highly conserved in non-human primates. The exact nucleic acid sequence deleted in human *KIR3DX1* depends on the precise position within exon 5 at which the start and end of the deletion is assumed to occur. In more detail, human *KIR3DX1* comprises an adenine adjacent to the deletion within exon 5 that is not found in other primate species.

If, as shown in Fig. 1, it is assumed that this adenine nucleotide is located immediately downstream of the deletion, the deleted nucleic acid sequence has the consensus sequence GAGTGGT. If, in contrast, this adenine is assumed to immediately precede the deletion, the consensus sequence deleted in human wild type *KIR3DX1* would be AGTGGTC. Therefore, in a preferred embodiment, the nucleic acid encoding the polypeptides, fusion proteins and receptors disclosed herein comprises a consensus sequence that is either GAGTGGT or AGTGGTC or a nucleotide sequence which is complementary or which hybridizes to any of said consensus sequences. Most preferably, the nucleic acid sequence comprises the consensus sequence GAGTGGT. It may also comprise an alternative nucleotide sequence of the same length, e.g., a sequence that encodes the same amino acids due to the redundancy of the genetic code. Optionally, the nucleic acid may be codon-optimized for expression in a human cell.

Preferably, the nucleic acid may encode a polypeptide of SEQ ID NO: 1 and thus, optionally, comprise a nucleic acid sequence corresponding to SEQ ID NO: 21.

In another embodiment, the nucleic acid encodes a polypeptide comprising both D2rep (SEQ ID NO: 1) and the KIR3DX1 D0 domain (SEQ ID NO: 5). Such nucleic acid may, e.g., comprise a nucleic acid sequence of SEQ ID NO: 22. It may also encode a polypeptide comprising KIR3DX1 D2rep (SEQ ID NO: 1) and D1 (SEQ ID NO: 6). Said nucleic acid may, e.g., comprise a nucleic acid sequence of SEQ ID NO: 23. In a further embodiment, the nucleic acid encodes the entire extracellular domain of KIR3DX1rep comprising all three of D0, D1 and D2rep (corresponding to an amino acid sequence of SEQ ID NO: 7). The nucleic acid may thus also comprise a nucleic acid sequence of SEQ ID NO: 24.

The nucleic acid may also further encode any of the herein disclosed fusion proteins, i.e., a polypeptide according to the invention or a polypeptide comprising any of SEQ ID NO: 15, 16, 17, 36 or 60 fused to an immunoglobulin Fc domain, e.g., an IgG Fc domain or linked to at least one additional antigen-binding moiety capable of binding, e.g., to CD16 or CD3. It may, e.g., encode any of the above-mentioned fusion proteins comprising an IgG1 Fc domain to obtain a polypeptide capable of inducing ADCC of KIR3DL2-expressing cells. Such a nucleic acid may therefore comprise a nucleic acid sequence of, e.g., any of SEQ ID NO: 25 (D2rep-Fc), 26 (D2rep+D0-Fc), 27 (D2rep+D1-Fc) or 28 (D2rep-D0+D1-Fc).

A nucleic acid according to the invention may also encode any of the herein disclosed KIR family receptor proteins comprising D2rep as part of their extracellular domains. Preferably, the nucleic acid encodes a full-length KIR3DX1rep-receptor according to SEQ ID NO: 13 and may thus comprise a nucleic acid sequence of SEQ ID NO: 29. It may also encode a chimeric KIR receptor according to SEQ ID NO: 31, e.g., comprising a nucleic acid sequence of SEQ ID NO: 32.

Also disclosed is a nucleic acid encoding a CAR construct as described herein. The nucleic acid may thus encode the herein described preferred CAR according to SEQ ID NO: 14, e.g., by comprising a nucleic acid sequence corresponding to SEQ ID NO: 30.

The nucleic acid disclosed herein is preferably provided as an expression vector suitable for being expressed in a human host cell, in particular, a human lymphoid host cell such as an NK cell, an NKT cell, a T cell or an innate lymphoid cell (ILC) of the ILC1, ILC2, or ILC3 type.

It may, e.g., be a viral vector, or a non-viral vector, e.g., a transposon, a vector suitable for CRISPR/CAS based recombination or a plasmid suitable for *in vitro* RNA transcription. If the vector consists of DNA, it preferably further comprises at least a regulatory region of a gene. The regulatory region may be a transcriptional regulatory region, e.g., selected from the group consisting of a promoter, an enhancer, a silencer, a locus control region, and a border element. Promoters or other expression control regions can be operably linked with the nucleic acid encoding the inventive polypeptide, fusion protein or receptor or to a nucleotide sequence which is complementary to or which hybridizes to said nucleotide sequence, e.g., to regulate expression of the polypeptide/fusion protein/receptor in a quantitative or in a tissue-specific manner. The promotor can be a native or, preferably, a heterologous promoter. The selection of promoters includes, e.g., strong, weak, inducible, tissue-specific and developmental-specific promoters. The promoter can be a non-viral promoter or a viral promoter. Preferably, it is a heterologous promoter, i.e., a promoter not naturally linked to KIR3DX1 in human NK cells or T cells, such as a long terminal repeat promoter, which is suitable for expression in human T cells. The inventive recombinant expression vectors can be designed for either transient expression, for stable expression, or for both. Also, the recombinant expression vectors can be made for constitutive expression or for inducible expression.

Furthermore, the vector comprising the nucleic acid of the invention may include one or more marker genes, which allows for selection or detection (e.g. via a marker gene expressing GFP) of transduced or transfected hosts.

### Cells

The present invention further provides a cell comprising a nucleic acid according to the invention. Preferably, the cell expresses any of the inventive receptor constructs disclosed herein, e.g., a full-length KIR3DX1rep-receptor or any of the chimeric KIR or CAR constructs disclosed herein from a nucleic acid according to the invention. Also disclosed is a human cell that expresses a full-length KIR3DX1 receptor of chimpanzee (SEQ ID NO: 33), gorilla (SEQ ID NO: 34), orangutan (SEQ ID NO: 35), bonobo (SEQ ID NO: 38) or siamang (SEQ ID NO: 62).

The cell preferably is a lymphoid cell, in particular a human lymphoid cell. It may be, e.g., a natural killer (NK) cell, a CD8⁺ cytotoxic T cell, a natural killer T cell (NKT cell) or a precursor thereof. NK cells are cells of the innate immune system that can recognize and rapidly kill target cells, in particular malignant and virus-infected cells even in the absence of antibodies and MHC class I proteins. NK cells are characterized by the expression of neural cell adhesion molecule (NCAM or CD56) and many express the FcγRIII receptor CD16. Cytotoxic T cells normally express T cell receptors that recognize a specific antigen presented by class I MHC proteins on the surface of a target cell upon which the T cell becomes activated and kills the antigen presenting cell. Cytotoxic T cells are characterized by the expression of the glycoprotein CD8, which assists in the interaction of the TCR with MHC class I. NKT cells share properties of both NK cells and cytotoxic T cells and can both kill target cells and release different cytokines upon activation. The cell may also be a CD4+ T helper cell, which may secrete cytokines that may sustain the immune response. It may also be a B cell, e.g., a B cell secreting an Fc fusion protein of the invention. It may also be an Innate Lymphoid Cell (ILC) of the ILC1, ILC2, or ILC3 type. Innate lymphoid cells (ILCs) are derived from common lymphoid progenitors (CLPs) and are typically found in both lymphoid (immune associated), and non- lymphoid tissues as well as rarely in the blood. In response to pathogenic tissue damage, ILCs contribute to immunity via the secretion of signalling molecules, and the regulation of both innate and adaptive immune cells (https://en.wikipedia.org/wiki/Innate_lymphoid cell). ILCs are divided into five distinct groups: NK cells, lymphoid tissue inducer (LTi) cells as well as ILC of groups 1-3.

Preferably, the obtained cell is a human lymphoid cell comprising a nucleic acid of the invention, and it is able to express any of the receptors disclosed herein on its surface. Accordingly, the cell is able to target KIR3DL2-presenting cells and consequentially to induce their specific killing by exerting cytotoxic activity or to release cytokines that may either promote or decrease activities of other immune cells. They may thus be utilized for immunotherapies against KIR3DL2-associated diseases as described in more detail below.

For treatment of humans, the cell is a human cell. Preferably, the cell is a cell isolated from a human, e.g., a human patient, in particular, a patient who is to be treated with the cell once it has been modified to express a receptor of the invention. Alternatively, the cell may be from a third-party donor who may be related or unrelated to the patient. Cells may be genetically engineered, e.g., in multiple ways. For example, in case the cell is a T cell, the cell may be genetically modified by knockout of the endogenous TCR and/or, in particular, if the cell is a heterologous cell, MHC class I. The lymphoid cells may also be generated from autologous or third-party donor stem cells, e.g., iPSC. Optionally, the stem cells are not human embryonic stem cells. The cell may also be derived from a suitable cell line such as NK cell lines NK-92, NKL, YT, NK3.3, or from a T cell line such as Jurkat.

### Pharmaceutical compositions

The present invention further provides a pharmaceutical composition comprising any of the herein disclosed (fusion-)polypeptides, nucleic acids or cells as well as mixtures thereof.

For instance, the pharmaceutical composition may comprise D2rep. The pharmaceutical composition may also comprise a polypeptide comprising D2rep and a D0 domain of human KIR3DX1 comprising or consisting of an amino acid sequence having at least 70% of SEQ ID NO: 5 and/or a D1 domain of KIR3DX1 comprising or consisting of an amino acid sequence having at least 70%, of SEQ ID NO: 6. Given that the D2 domains of chimpanzee, gorilla, orangutan, bonobo or siamang KIR3DX1 are able to bind to human KIR3DL2 as well, in some embodiments, the pharmaceutical composition may alternatively comprise a polypeptide comprising an amino acid sequence of to any of SEQ ID NO: 15, 16, 17, 36 or 60, respectively.

In further embodiments, the pharmaceutical composition comprises any of the aforementioned fusion proteins comprising at least D2rep or a D2 domain according to SEQ ID NO: 15, 16, 17, 36 or 60 fused to an Fc domain of an immunoglobulin, e.g., an IgG1 or an IgG4 Fc domain, or linked to at least one additional antigen-binding moiety, as described herein. Accordingly, in some embodiments, the present invention provides a pharmaceutical composition comprising a polypeptide as disclosed herein capable of inducing ADCC in a KIR3DL2-expressing cell.

The pharmaceutical composition may also comprise a nucleic acid that, when being expressed in a cell of a subject, gives rise to any of the herein disclosed polypeptides of the invention. For instance, the pharmaceutical composition may comprise an expression vector as described herein such as, e.g., a viral vector directed to a target cell that, when being expressed by said targeted cell, results in the generation of a polypeptide of the invention that may be secreted.

In other embodiments, the pharmaceutical composition may comprise a cell as disclosed herein, e.g., a cytotoxic lymphocyte such as an NK cell or a cytotoxic T cell expressing any of the KIR-family or CAR receptors of the present invention. For instance, the pharmaceutical composition may comprise an NK cell, an NKT cell or cytotoxic T cell expressing a CAR of SEQ ID NO: 14, a full-length human KIR3DX1rep receptor corresponding to SEQ ID NO: 13 or a chimeric KIR receptor of SEQ ID NO: 31 from a corresponding nucleic acid of the present invention. The pharmaceutical composition may also comprise a cell, e.g., a lymphoid cell such as a natural killer (NK) cell, a CD8⁺ cytotoxic T cell, an NKT cell or a precursor thereof expressing a KIR3DX1 receptor of chimpanzee (SEQ ID NO: 33), gorilla (SEQ ID NO: 34), orangutan (SEQ ID NO: 35), bonobo (SEQ ID NO: 38) or siamang (SEQ ID NO: 62).

The pharmaceutical composition according to the invention may comprise additional ingredients such as, e.g., a pharmaceutically acceptable excipient. The term "pharmaceutically acceptable" refers those compounds, materials, compositions, and/or dosage forms that are, within the scope of sound medical judgement, suitable for use in contact with the tissues of the subject without causing excessive toxicity, irritation, allergic response or other problems or complications commensurate with a reasonable benefit/risk ratio.

In many embodiments, the pharmaceutical composition further comprises a suitable carrier, i.e., a compound, composition, structure or substance that, when combined with the polypeptides or cells described herein, aids or facilitates preparation, storage, administration, delivery, effectiveness, selectivity, or any other feature of the peptides or cells for its intended use or purpose. It may, e.g., comprise water, a saline, e.g., physiological saline, or a buffer.

The pharmaceutical composition may also comprise additional therapeutically active ingredients or agents that may be used to fight a disease of interest. For instance, in addition to a polypeptide of the invention, the pharmaceutical composition may comprise, e.g., an anticancer drug such as a chemotherapeutic drug. The present invention also covers pharmaceutical compositions in which a polypeptide, fusion protein or receptor described herein serves as a targeting moiety to target another therapeutically active agent, e.g., an anti-cancer drug, to a cell or a tissue expressing KIR3DL2. In such a scenario, the other therapeutically active agent may be encapsulated as a cargo in a suitable vector, such as, e.g., a lipid vesicle, a lipid nanoparticle, a bacterial minicell or a polymeric micro- or nanoparticle, wherein the vector carries a polypeptide, fusion protein or receptor according to the invention on its surface or is linked to it. The polypeptide, fusion protein or receptor may then recruit the vector and thus the additional therapeutically active agent to a target cell or tissue where the agent is released to exert its therapeutic effect.

### Therapeutic uses and detection methods

The present invention is further directed to uses of the herein disclosed polypeptides, fusion proteins, cells and pharmaceutical compositions in methods of treating a disease or condition associated with KIR3DL2 expression.

In the context of the invention, treating a disease or condition is to be understood to comprise a curative medical therapy of a subject with the intent to cure, ameliorate or stabilize a condition. The term "condition" refers to a disease, a syndrome, a disorder or a particular physiological state of an organism that is either manifested by distinct symptoms or can be diagnosed by using established markers capable of recognizing said state. In some embodiments, treating a disease or condition is intended to mean that the progression of the disease or condition is to be slowed, stopped or, preferably, reversed to allow for a perceivable improvement of the subject's well-being and health. The subject that is to be treated preferably is a human.

For instance, the herein disclosed polypeptides, fusion proteins, cells or pharmaceutical compositions may be used in a method of treating of a rheumatic disease in a subject, in particular a rheumatic disease associated with HLA-B*27. The present invention thus also provides a method of treating a rheumatic disease in a subject, which comprises a step of administering any of the polypeptides, fusion proteins, cells or pharmaceutical compositions to a subject in need thereof.

A rheumatic disease is a condition associated with inflammation and chronic, often intermittent pain affecting the joints, tendons, muscles, ligaments and/or bones. Arthritis is a common form of rheumatic disease affecting the joints. Preferably, the rheumatic disease that is to be treated using any of the herein disclosed polypeptides, cells or pharmaceutical compositions is associated with HLA-B*27. It may be a form of arthritis, most preferably a type of spondyloarthritis (SpA), such as ankylosing spondylitis (AS).

The term spondyloarthrides (SpA) relates to a group of chronic inflammatory arthritic disorders affecting the vertebral column or spine. They are characterized by axial and peripheral arthritis, extraskeletal manifestations including uveitis, and an extremely strong association with human leukocyte antigen HLA-B*27 (Bowness et al., 2011). AS, also known under the names Bechterew's disease or Morbus Bechterew, is the most common form of SpA and refers to an autoimmune disease affecting the joints of the spine in particular where the spine joins the pelvis. 94% of AS patients are HLA-B*27 positive (Bowness et al., 2011).

The molecular mechanism of AS development is thought to involve two steps in HLA-B*27-positive patients: the first step is the induced expression of KIR3DL2 on activated CD4⁺ T helper cells (Th cells), and the second step is the molecular interaction of KIR3DL2 with HLA-B*27, which licenses CD4⁺ Th cells to develop into inflammatory Th17 cells expressing increased amounts of the inflammatory cytokine IL-17 and reduced amounts of IL-2 (Chan et al., 2005; Bowness et al., 2011; Ridley et al., 2016; Wong-Baeza et al., 2013).

As described herein, the present inventors found that repaired human KIR3DX1 can bind to the D0 domain of KIR3DL2 via its D2rep domain. Accordingly, the herein disclosed polypeptides and fusion proteins comprising at least D2rep as defined herein may interfere with the interaction of HLA-B*27 and KIR3DL2 on the surface of CD4⁺ Th cells and thereby prevent the CD4⁺ Th cells from developing into Th17 cells. In case the polypeptides of the invention are provided in the form of multispecific engagers capable of engaging NK cell or T cell-mediated cytotoxicity, as described herein, or as fusion proteins comprising an immunoglobulin Fc domain capable of inducing ADCC in a target cell, such as, e.g., an IgG1-Fc domain, the use of these polypeptides can also directly trigger the killing of the CD4+ Th cells expressing KIR3DL2. Death of CD4+ Th cells can also be achieved by administering to the subject a cytotoxic lymphocyte expressing a KIR or CAR receptor, as disclosed herein, that recognizes KIR3DL2 on the surface of the CD4⁺ Th cells.

The polypeptides, fusion proteins, cells and pharmaceutical compositions according to the invention may also be used to interfere with the interaction of KIR3DL2 and unmethylated CpG oligonucleotides derived from bacterial DNA thereby attenuating the TLR9-mediated inflammatory response of KIR3DL2-positive cells towards microbial infection. As already explained, once the KIR3DL2 binds to this PAMP via its D0 domain, it is endocytosed and loads the CpG cargo on Toll-like receptor 9 (TLR9), which drives the expression of IFN-y and causes elevated cytotoxicity in the KIR3DL2-expressing cells (Sivori et al., 2010a). Thus, in some embodiments, the herein disclosed polypeptides, fusion proteins, cells or pharmaceutical compositions may also be for use in treating or even preventing the excessive TLR9-mediated inflammatory response associated with sepsis or arising in response to superinfections, secondary infections superimposed on an earlier first one, especially by a different microbial agent of exogenous or endogenous origin, that is resistant to the treatment being used against the first infection.

Since the D2 domain of chimpanzee, gorilla, orangutan, bonobo and siamang can bind to the D0 domain of human KIR3DL2 and may thus block its interaction with ligands, a polypeptide comprising a sequence according to any of SEQ ID NO: 15, 16, 17, 36 or 60 can also be used to treat any of the herein disclosed rheumatic diseases or the excessive TLR9-mediated inflammatory response associated with sepsis or superinfections.

Moreover, given that KIR3DL2 was also found to be expressed on the surface of malignant T cells in various T-cell leukemias, another aspect of the invention is the use of the herein disclosed fusion proteins capable of inducing ADCC, cells or pharmaceutical compositions in a method of treating a cancer expressing KIR3DL2, wherein the cancer expressing KIR3DL2 may be, e.g., cutaneous T-cell lymphoma (CTCL), Sézary syndrome, mycosis fungoides, CD30⁺ cutaneous lymphoma or adult T-cell leukemia (ATL). Accordingly, the present invention also discloses a method of treating a cancer expressing KIR3DL2, wherein the method comprises administering to a subject in need thereof any of the herein described fusion proteins, cells or pharmaceutical compositions.

In particular, human lymphoid cells, such as NK cells, NKT cells or CD8+ cytotoxic lymphocytes expressing the herein disclosed KIR constructs or CAR constructs may be used in immunotherapy to treat T cell lymphomas associated with KIR3DL2 expression. KIR3DL2-expressing cancer cells may also be killed by administering any of the herein disclosed ADCC- inducing fusion proteins or pharmaceutical compositions comprising the same.

Cutaneous T-cell lymphoma (CTCL) refers to a heterogeneous group of non-Hodgkin lymphomas caused by a mutation of T cells. The cancerous T cells migrate to the skin, resulting in lesions that initially resemble an itchy rash before transforming into larger plaques and tumors that may ultimately spread to other tissues. Sézary syndrome and mycosis fungoides represent the two most common types of CTCL. CD30⁺ cutaneous lymphoma, also known as primary cutaneous anaplastic large-cell lymphoma, is another type of CTCL characterized by solitary or localized skin lesions that have a tendency to ulcerate. Additional types of CTCL that may be treated with the herein disclosed polypeptides, cells or pharmaceutical compositions include, e.g., Pagetoid reticulosis, Granulomatous slack skin, Lymphomatoid papulosis, Pityriasis lichenoides chronica, Pityriasis lichenoides et varioliformis acuta, Secondary cutaneous CD30+ large cell lymphoma, Non-mycosis fungoides CD30- cutaneous large T-cell lymphoma, Pleomorphic T-cell lymphoma, Lennert lymphoma, Subcutaneous T-cell lymphoma, Angiocentric lymphoma or Blastic NK-cell lymphoma. ATL is a rare yet highly aggressive form of non-Hodgkin lymphoma that is caused by infection with the human T cell leukemia/lymphotropic virus 1 (HTLV-1). The disease is associated with symptoms such as hypercalcemia, skin lesions, and lytic bone lesions.

Increased expression of KIR3DL2 has further been described in myelodysplastic syndrome, i.e., a group of malignant disorders of hematopoiesis. Accordingly, the herein disclosed fusion proteins, cells or pharmaceutical compositions may also be used for treating myelodysplastic syndrome.

In another aspect, the present invention relates to different diagnostic methods for detecting KIR3DL2 expression in a subject or a sample isolated from the subject by using a polypeptide or fusion protein as disclosed herein as a capture or detecting agent. This way it is possible to diagnose whether a subject suffers from a disease associated with elevated KIR3DL2 expression, as described herein.

The methods preferably are in vitro methods and rely on the use of a polypeptide or fusion protein of the present invention that is able to bind and thus detect KIR3DL2 protein in a sample obtained from a subject. The method may comprise contacting a sample obtained from a subject with a polypeptide of fusion protein of the invention or a polypeptide comprising an amino acid sequence of any of SEQ ID NO: 15, 16, 17, 36 or 60, wherein the polypeptide or the fusion protein preferably is linked to a label. Binding can then be detected.

For instance, in one embodiment, the detection method comprises the step of performing an enzyme-linked immunosorbent assay (ELISA) during which a liquid sample obtained from a subject is brought into contact with a polypeptide or a fusion protein according to the present invention or a polypeptide comprising an amino acid sequence of any of SEQ ID NO: 15, 16, 17, 36 or 60 to detect soluble KIR3DL2.

Preferably, the liquid sample is or comprises a body fluid isolated from the subject, e.g., the sample may be or may comprise any body fluid selected from the group comprising blood, lymph, saliva, urine, cerebrospinal fluid, amniotic fluid, ejaculate or vaginal fluid. Preferably, the body fluid is blood. The sample may also be or comprise a mixture of different body fluids, e.g., a mixture of blood and lymph. In the context of the invention, the term "liquid sample" also encompasses preparations of a body fluid. Thus, the sample may also be or comprise a body fluid that has been processed before being contacted with a polypeptide of the invention in the ELISA, e.g., the body fluid may be diluted, concentrated, filtered, centrifuged or otherwise cleared of certain ingredients such as, e.g., cellular components. Accordingly, if the body fluid to be analyzed is blood, this also includes preparations thereof, such as plasma or serum. The liquid sample may further comprise additional ingredients, e.g., the body fluid contained therein may be mixed with water or a suitable buffer solution.

ELISA refers to an analytical assay in which the presence of a ligand of interest in a liquid sample is detected by one or more detection agents such as, e.g., antibodies, wherein at least one of these detection agents is linked to an enzyme such as horseradish peroxidase that can convert an added substrate into a detectable signal. The skilled person will have no difficulty to design an ELISA that uses a polypeptide of the present invention as a detection agent for detecting KIR3DL2. For instance, the ELISA may be a direct ELISA or a sandwich ELISA, preferably a sandwich ELISA.

Briefly, a direct ELISA involves steps in which the liquid sample comprising KIR3DL2, e.g., a body fluid or preparation thereof, is added into a reaction vessel such as a well of a microtiter plate where the KIR3DL2 is allowed to adhere to the surface of said reaction vessel. A polypeptide according to the invention comprising the amino acid sequence of D2rep or a polypeptide comprising an amino acid sequence of the chimpanzee, gorilla, orangutan or bonobo KIR3DX1 D2 domain according to any of SEQ ID NO: 15, 16, 17 or 36, respectively, is conjugated to an enzyme such as horseradish peroxidase to specifically bind to the KIR3DL2 coating the reaction vessel. The enzyme conjugated to the polypeptide then converts an added substrate into a detectable signal. For instance, in case the enzyme is horseradish peroxidase, the substrate may be a chromogenic substrate such as, e.g., 3,3',5,5'Tetramethylbenzidine (TMB), 3,3'-Diaminobenzidine (DAB), 2,2'-azino-bis(3-ethylbenzothiazoline-6-sulfonic acid (ABTS) or o-phenylenediamine dihydrochloride (OPD), which upon enzymatic conversion, results in a colour change in the reaction vessel. The intensity of the colour change can optionally be quantified using a suitable spectrometer.

However, in a preferred embodiment, the ELISA that is to be performed in the herein disclosed detection method is a sandwich-ELISA. For this, a polypeptide according to the invention or a polypeptide comprising an amino acid of any of SEQ ID NO: 15, 16, 17, 36 or 60 may serve as a "capture agent" which is attached to the surface of a solid substate, typically, the reaction vessel, e.g., the wells of a 96-well microtiter plate. In case the liquid sample added to the wells comprises soluble KIR3DL2, the KIR3DL2 will be thus captured by the attached polypeptides. Subsequent washing removes all unbound and thus unwanted antigens. A second "detection agent", e.g., a primary antibody recognizing another epitope of KIR3DL2, is added so that the KIR3DL2 is "sandwiched" between the polypeptide attached to the reaction vessel and the detection agent. Of note, in other embodiments, the polypeptide of the invention or a polypeptide according to SEQ ID NO: 15, 16, 17, 36 or 60 may also be used as detection agent that is added after the KIR3DL2 has been captured by another capture agent, e.g., an antibody attached to the reaction vessel. The detection agent may either be directly conjugated to an enzyme such as horseradish peroxidase, or another conjugated secondary antibody is added to quantify the result.

In another embodiment, the herein disclosed polypeptides or fusion proteins may be used to detect and, optionally, sort cells expressing KIR3DL2 on their surface, e.g., by microscopy or with Fluorescence activated cell sorting (FACS). The polypeptides or fusion proteins may be labelled, e.g., with a fluorescent label. Alternatively, they may be indirectly labelled.

Accordingly, the present invention also discloses an in-vitro method for detecting KIR3DL2 expression in a subject comprising steps of
i) contacting a sample comprising cells obtained from a subject with a polypeptide or fusion protein of the invention or a polypeptide comprising an amino acid sequence of SEQ ID NO: 15, 16, 17, 36 or 60, wherein the polypeptide or the fusion protein further comprises a label, preferably a fluorescent label; and
ii) detecting cells expressing KIR3DL2 on their surface, e.g., by flow cytometry.

In summary, the present invention is based on the hitherto completely unexpected finding that the repaired D2 domain of human KIR3DX1, D2rep, can bind to the human KIR3DL2 receptor. Polypeptides comprising the D2rep domain of human KIR3DX1 can therefore be used both as a diagnostic detection agent and, in various forms, as a therapeutic agent to combat diseases and conditions associated with KIR3DL2 expression.

Throughout the invention, the term "about" is intended to be understood as "+/- 10%". If "about" relates to a range, it refers to both lower and upper limits of the range. "A" is intended to mean "one or more", if not explicitly mentioned otherwise.

All literature cited herein is herewith fully incorporated. The present invention is further illustrated, but not limited, by the following examples.

**Table 1: List of Sequences**

| | |
|---|---|
| SEQ ID NO: 1 | AA of human KIR3DX1 D2rep |
| SEQ ID NO: 2 | Consensus 13-AA sequence missing in wild type human KIR3DX1 D2 domain |
| SEQ ID NO: 3 | 13-AA sequence missing in wild type human KIR3DX1 D2 domain |
| SEQ ID NO: 4 | 13-AA sequence from chimpanzee and gorilla corresponding to the 13-AA sequence missing in wild type human KIR3DX1 D2 domain |
| SEQ ID NO: 5 | D0 domain of human KIR3DX1 (AA) |
| SEQ ID NO: 6 | D1 domain of human KIR3DX1 (AA) |
| SEQ ID NO: 7 | Extracellular domain of human KIR3DX1rep comprising D0, D1 and D2rep (AA) |
| SEQ ID NO: 8 | IgG1-Fc region (AA) |
| SEQ ID NO: 9 | IgG4-Fc region (AA) |
| SEQ ID NO: 10 | KIR3DX1rep stem region (AA) |
| SEQ ID NO: 11 | KIR3DX1rep transmembrane region (AA) |
| SEQ ID NO: 12 | KIR3DX1rep cytoplasmic region (AA) |
| SEQ ID NO: 13 | Full-length KIR3DX1rep receptor (AA) |
| SEQ ID NO: 14 | KIR3DX1rep CAR construct (AA) |
| SEQ ID NO: 15 | Chimpanzee KIR3DX1 D2 domain (AA) |
| SEQ ID NO: 16 | Gorilla KIR3DX1 D2 domain (AA) |
| SEQ ID NO: 17 | Orangutan KIR3DX1 D2 domain (AA) |
| SEQ ID NO: 18 | Chimpanzee KIR3DX1 extracellular domain (AA) |
| SEQ ID NO: 19 | Gorilla KIR3DX1 extracellular domain (AA) |
| SEQ ID NO: 20 | Orangutan KIR3DX1 extracellular domain (AA) |
| SEQ ID NO: 21 | D2rep (NA) |
| SEQ ID NO: 22 | D2rep + D0 (NA) |
| SEQ ID NO: 23 | D2rep + D1 (NA) |
| SEQ ID NO: 24 | Extracellular domain of human KIR3DX1rep (NA) |
| SEQ ID NO: 25 | D2rep-Fc (NA) |
| SEQ ID NO: 26 | D2rep+D0-Fc (NA) |
| SEQ ID NO: 27 | D2rep+D1-Fc (NA) |
| SEQ ID NO: 28 | D2rep-D0+D1-Fc (NA) |
| SEQ ID NO: 29 | Full-length KIR3DX1rep receptor (NA) |
| SEQ ID NO: 30 | FLAG-tagged KIR3DX1rep CAR construct (NA) |
| SEQ ID NO: 31 | chimeric KIR receptor comprising extracelluar part of KIR3DX1rep and transmembrane and cytoplasmic regions of human KIR3DS1 (AA) |
| SEQ ID NO: 32 | chimeric KIR receptor comprising extracelluar part of KIR3DX1rep and transmembrane and cytoplasmic regions of human KIR3DS1 (NA) |
| SEQ ID NO: 33 | KIR3DX1 receptor of chimpanzee (AA) |
| SEQ ID NO: 34 | KIR3DX1 receptor of gorilla (AA) |
| SEQ ID NO: 35 | KIR3DX1 receptor of orangutan (AA) |
| SEQ ID NO: 36 | Bonobo KIR3DX1 D2 domain (AA) |
| SEQ ID NO: 37 | Bonobo KIR3DX1 extracellular domain (AA) |
| SEQ ID NO: 38 | KIR3DX1 receptor of Bonobo (AA) |
| SEQ ID NO: 39 | 13-AA sequence from orangutan and siamang corresponding to the 13-AA sequence missing in wild type human KIR3DX1 D2 domain |
| SEQ ID NO: 40 | Partial exon 5 sequence of Homo sapiens KIR3DX1 gene (Fig. 1) |
| SEQ ID NO: 41 | Partial exon 5 sequence of Pan troglodytes KIR3DX1 gene (Fig. 1) |
| SEQ ID NO: 42 | Partial exon 5 sequence of Gorilla gorilla KIR3DX1 gene (Fig. 1) |
| SEQ ID NO: 43 | Partial exon 5 sequence of Pongo abelii KIR3DX1 gene (Fig. 1) |
| SEQ ID NO: 44 | Partial exon 5 sequence of N. leukogenys KIR3DX1 gene (Fig. 1) |
| SEQ ID NO: 45 | Partial exon 5 sequence of Macaca leonina KIR3DX1 gene (Fig. 1) |
| SEQ ID NO: 46 | Partial exon 5 sequence of Papio anubis KIR3DX1 gene (Fig. 1) |
| SEQ ID NO: 47 | Partial exon 5 sequence of Colobus angolensis KIR3DX1 gene (Fig. 1) |
| SEQ ID NO: 48 | Partial exon 5 sequence of Theropitecus gelada KIR3DX1 gene (Fig. 1) |
| SEQ ID NO: 49 | Partial exon 5 sequence of Macaca mulatta KIR3DX1 gene (Fig. 1) |
| SEQ ID NO: 50 | Partial exon 5 sequence of Macaca fascicularis KIR3DX1 gene (Fig. 1) |
| SEQ ID NO: 51 | Partial exon 5 of Macaca nemestrina KIR3DX1 gene (Fig. 1) |
| SEQ ID NO: 52 | Partial exon 5 sequence of Cercocebus atys KIR3DX1 gene (Fig. 1) |
| SEQ ID NO: 53 | Partial exon 5 sequence of R. roxellana KI R3DX1 gene (Fig. 1) |
| SEQ ID NO: 54 | Partial exon 5 sequence of Rhinopithecus bieti KIR3DX1 gene (Fig. 1) |
| SEQ ID NO: 55 | Partial exon 5 sequence of T. francoisi KI R3DX1 gene (Fig. 1) |
| SEQ ID NO: 56 | Partial exon 5 sequence of Callithrix jacchus KIR3DX1 gene (Fig. 1) |
| SEQ ID NO: 57 | Partial exon 5 sequence of Saimiri boliviensis KIR3DX1 gene (Fig. 1) |
| SEQ ID NO: 58 | Partial exon 5 sequence of Carlito syrichta KIR3DX1 gene (Fig. 1) |
| SEQ ID NO: 59 | Partial exon 5 sequence of Microcebus murinus KIR3DX1 gene (Fig. 1) |
| SEQ ID NO: 60 | Siamang KIR3DX1 D2 domain (AA) |
| SEQ ID NO: 61 | Siamang KIR3DX1 extracellular domain (AA) |
| SEQ ID NO: 62 | KIR3DX1 receptor of Siamang (AA) |

### Figure Legends:

**Fig. 1****: Comparison of partial exon 5 sequences of KIR3DX1 from different primate species.** The consensus sequence in the region of the gap is indicated.

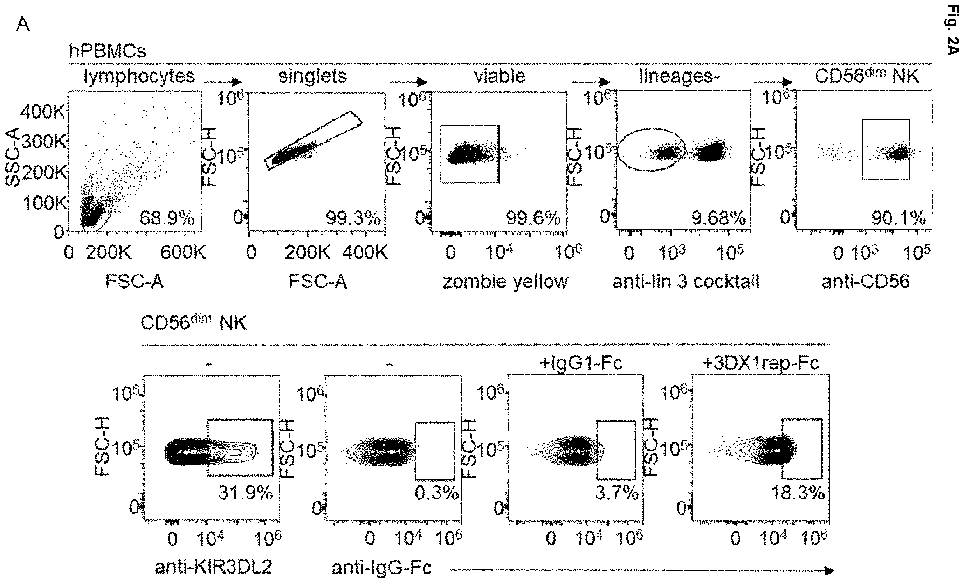

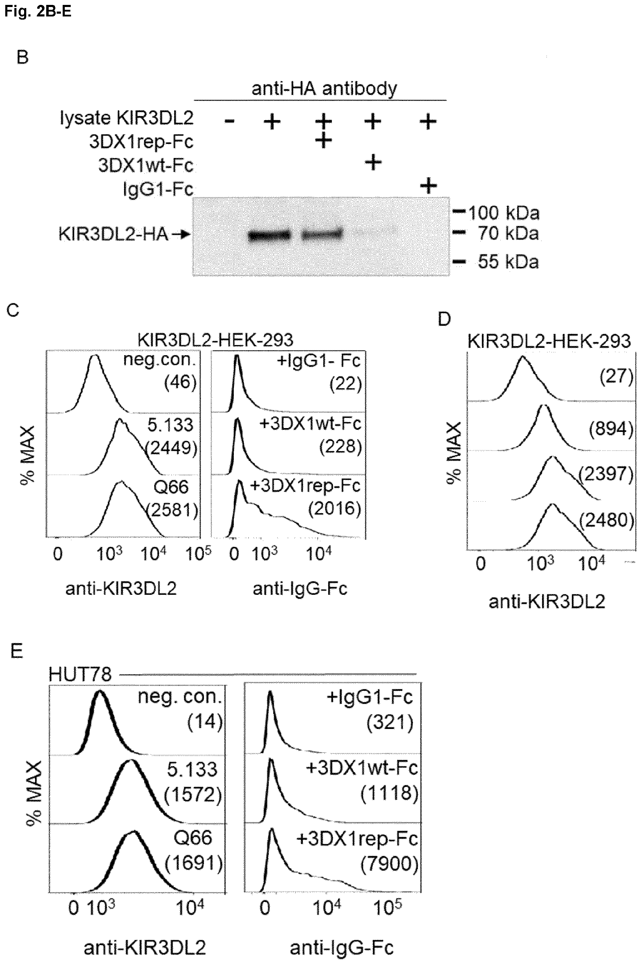

**Fig. 2****: Analysis of specific binding of repaired KIR3DX1 (KIR3DX1rep)-Fc protein to human KIR3DL2. A)** Upper panel shows the gating strategy to identify NK cells in PBMCs, lower panel shows percentage of KIR3DL2-positive NK cells using an anti-KIR3DL2 antibody, and percentage of anti-IgG-positive cells either without addition of any Fc protein (-), or addition of IgG1-Fc control protein (+lgG1-Fc) or addition of KIR3DX1rep-IgG1-Fc (+KIR3DX1rep-lgG1-Fc). **B)** Western blot analysis including lysate of HEK-293 cells either untransfected (-) or transfected with a construct for expression of HA-tagged KIR3DL2 (+) without adding any Fc proteins for co-immunoprecipitation (-), or adding different Fc proteins (+). Anti-HA tag antibody was used to detect KIR3DL2. Molecular weight markers are indicated. **C)** Flow cytometric analyses of KIR3DL2-expressing HEK-293 cells using anti-KIR3DL2 antibodies 5.133 and Q66 and an unstained control (neg. con.) and using anti-IgG-Fc antibody upon addition of either IgG1-Fc control protein, or KIR3DX1 wildtype-lgG1-Fc (KIR3DX1wt-lgG1-Fc), or KIR3DX1repaired-IgG1-Fc (KIR3DX1rep-IgG1-Fc). The median fluorescence intensities are indicated in brackets. **D)** Flow cytometric analyses of KIR3DL2-expressing HEK-293 cells. Cells were first incubated with either KIR3DXrep-IgG1-Fc (second uppermost curve), or with IgG1-Fc control protein (third uppermost curve), or with no IgG1-Fc protein (-, bottom curve) and subsequently incubated with the anti-KIR3DL2 antibody. Median fluorescence intensities are indicated in brackets. KIR3DX1rep-IgG1-Fc binding to KIR3DL2 impedes binding of the anti-KIR3DL2 antibody to its target protein KIR3DL2. Neg. con. Indicates no addition of anti-KIR3DL2 antibody (upper curve). **E)** Flow cytometric analyses of human cutaneous T cell lymphoma (Sézary syndrome) cell line HUT78 with anti-KIR3DL2 antibodies 5.133 and Q66 (left panel) and with anti-IgG-Fc antibody after incubation of the HUT78 cells with either IgG1-Fc control protein, or KIR3DX1 wildtype-lgG1-Fc protein, or KIR3DX1rep-IgG1-Fc protein (right panel). Median fluorescence intensities are indicated in brackets.

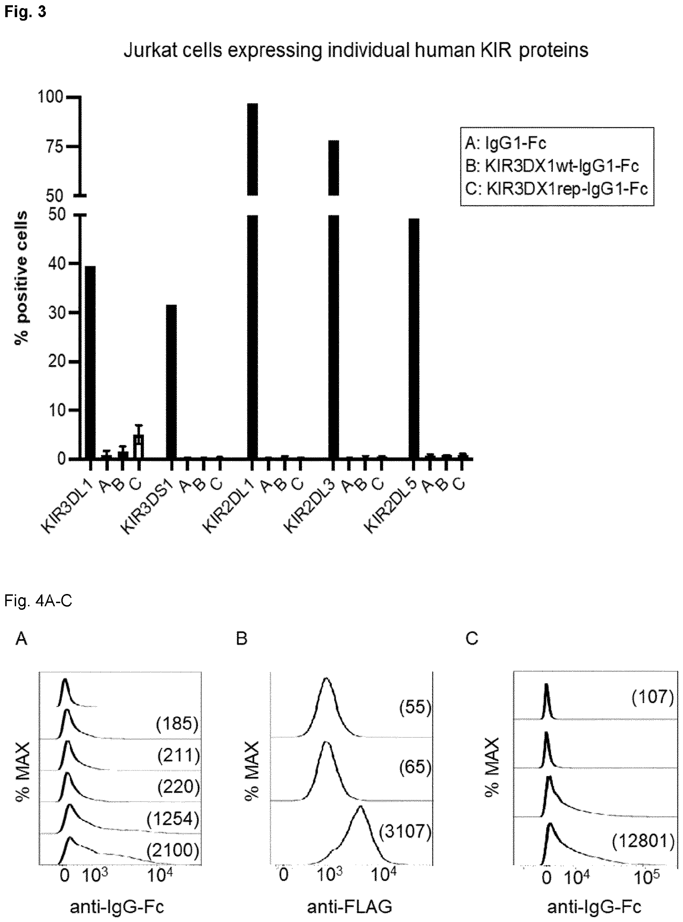

**Fig. 3****: Flow cytometric analyses of binding of KIR3DX1rep-lgG1-Fc to other human KIR proteins.** Expression of KIR3DL1, KIR3DS1, KIR2DL1, KIR2DL3, or KIR2DL5 in individually transfected Jurkat cells was analyzed with specific antibodies against the individual KIR proteins. Binding of a control IgG1-Fc protein (A), or wildtype KIR3DX1-Fc (B, KIR3DX1wt-lgG1-Fc) protein, or repaired KIR3DX1-Fc (C, KIR3DX1rep-IgG1-Fc) protein is shown as percentage positive cells. Binding of different Fc proteins was measured with an anti-IgG-Fc antibody.

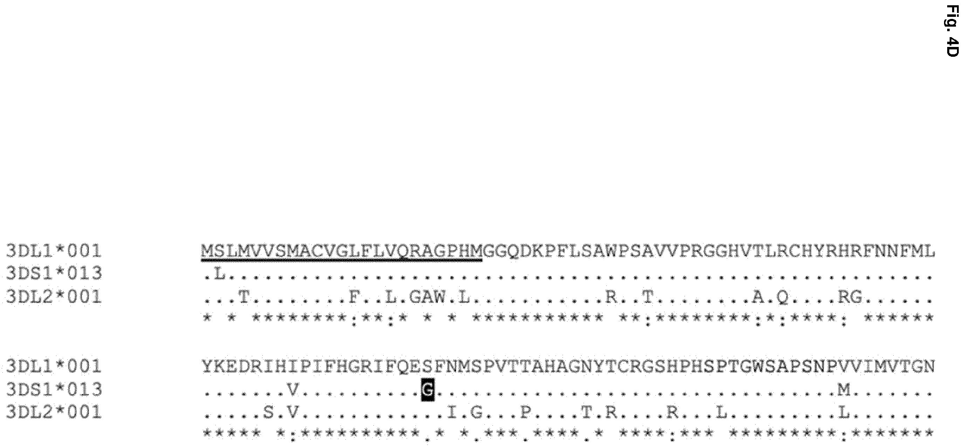

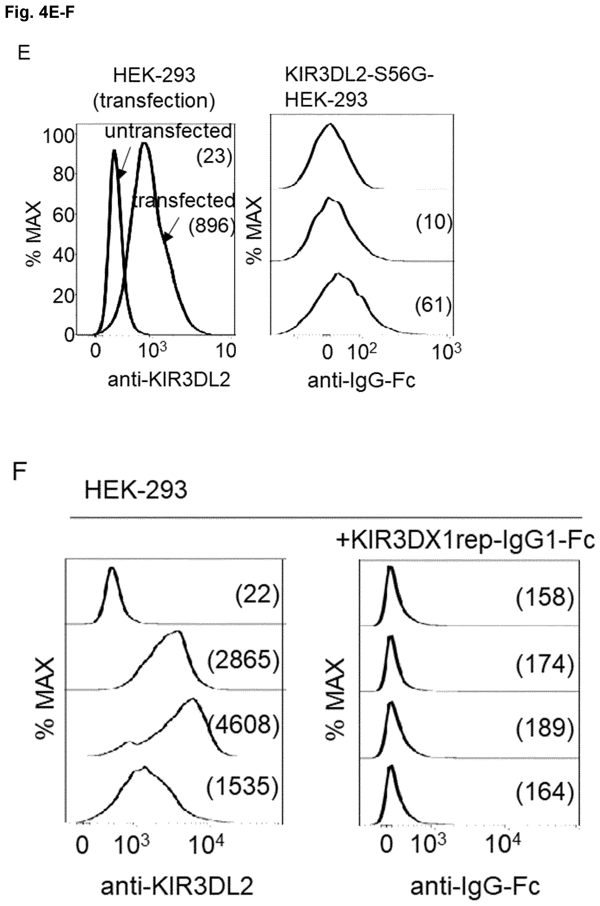

**Fig. 4****: Identification of sites involved in specific binding of KIR3DX1rep and KIR3DL2. A)** Flow cytometric analyses of KIR3DL2-expressing HEK-293 cells with anti-IgG-Fc upon addition of no further components (neg. con., uppermost curve), or IgG1-Fc as control protein (second uppermost curve), or the individual D0 (third uppermost curve), D1 (fourth uppermost curve), and D2 (fifth uppermost curve) domains of KIR3DX1rep as IgG1-Fc proteins, or the complete KIR3DX1rep-IgG1-Fc protein (bottom curve) as positive control. Median fluorescence intensities are indicated in brackets. **B)** Flow cytometric analyses of cell surface expression of FLAG-tagged KIR3DX1 using constructs for the expression of the complete coding regions of KIR3DX1 wildtype (KIR3DX1wt, middle curve), or KIR3DX1-repaired (KIR3DX1rep, bottom curve) with an anti-FLAG antibody in transfected HEK-293 cells. Untransfected HEK-293 served as negative control (neg. con., uppermost curve). Median fluorescence intensities are indicated in brackets. **C)** Flow cytometric analyses of FLAG-tagged full-length KIR3DX1-repaired expressed in HEK-293 cells with anti-IgG-Fc upon addition of IgG1-Fc (uppermost curve) as control protein, or the individual D0 (third uppermost curve), and D2 (second uppermost curve) domains of KIR3DL2 as IgG1-Fc proteins, or the complete KIR3DL2-lgG1-Fc protein (bottom curve) as positive control. Median fluorescence intensities are indicated in brackets. **D)** Amino acid comparison of the leader sequence (underlined) and D0 domain of KIR3DL1*001, KIR3DS1*013, and KIR3DL2*001. As KIR3DS01 does not bind KIR3DX1rep, amino acid position 56 (marked in black) in the D0 domain is the only amino acid position where KIR3DS1 is different from weakly binding KIR3DL1 and strongly binding KIR3DL2. **E)** Flow cytometric analyses of the specific interaction site in the D0 domain of KIR3DL2. HEK-293 cells transfected with a construct encoding KIR3DL2 with a point mutation at position 56 (glycine instead of a serine residue, S56G). Left panel shows the expression of KIR3DL2 -S56G analyzed with an anti-KIR3DL2 antibody. Right panel shows binding of the anti-IgG-Fc upon either no addition (neg. con., uppermost curve), or addition of IgG1-Fc (middle curve), or KIR3DX1rep-lgG1-Fc (bottom curve). This analysis demonstrates that amino acid position 56 in KIR3DL2 is involved in specific binding of KIR3DX1rep. **F)** Flow cytometric analyses of the effect of homo-dimerization of KIR3DL2 on binding of KIR3DX1rep. Left panel shows expression of KIR3DL2 in untransfected HEK-293 cells (untransfected, uppermost curve), or in HEK-293 cells transfected with either mutated KIR3DL2 at amino acid position 313 (C313P, second uppermost curve), or mutated at amino acid position 279 (C279Y, third uppermost curve), or mutated at both positions (C313P-C279Y, bottom curve). Right panel shows binding of anti-IgG-Fc upon addition of KIR3DX1rep-lgG1-Fc to either untransfected HEK-293 cells, or HEK-293 cells transfected with either mutated KIR3DL2 at amino acid position 313 (C313P),or mutated at amino acid position 279 (C279Y), or mutated at both positions (C313P-C279Y). The analysis demonstrates that although KIR3DL2 mutated at specific cysteine residues is expressed at the cell surface, the individual mutant KIR3DL2 proteins are no longer bound by KIR3DX1rep-IgG1-Fc. As the mutated KIR3DL2 cannot form homo-dimers, KIR3DX1rep-lgG1-Fc detects only the homodimeric form of KIR3DL2.

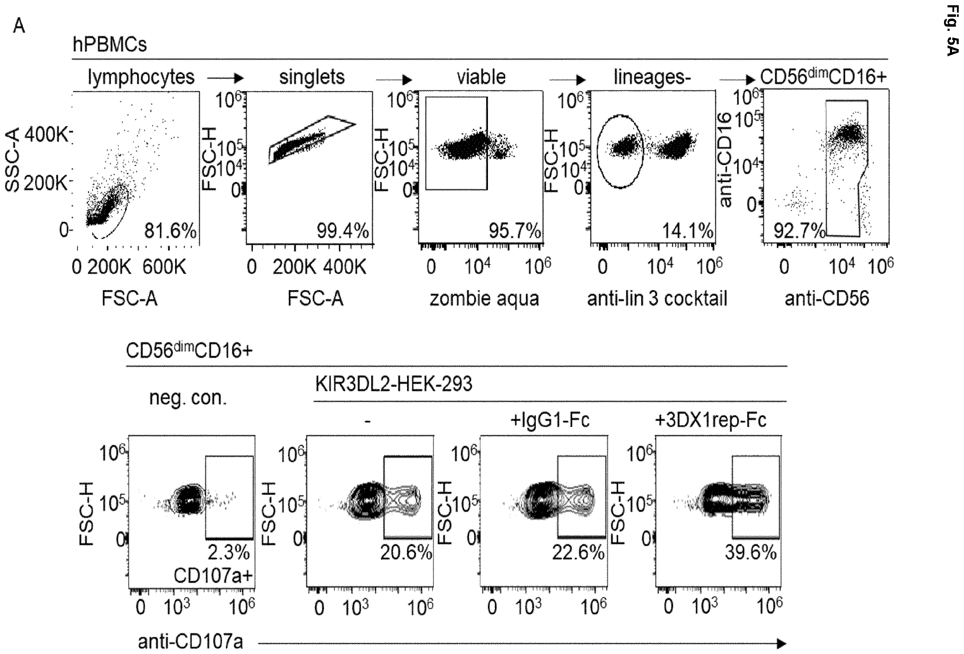

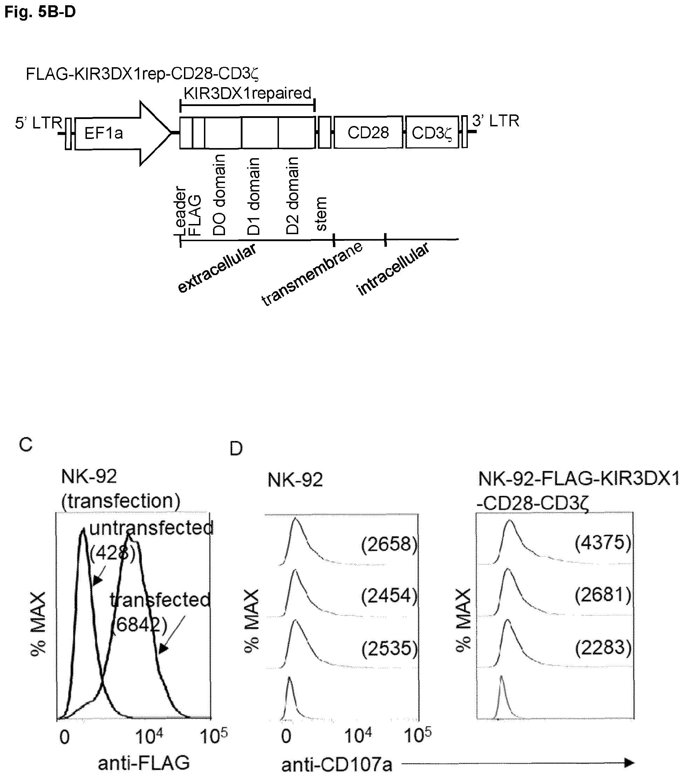

**Fig. 5****: KIR3DX1rep can be used to detect and destroy KIR3DL2-expressing cells. A)** Upper panel shows flow cytometric analysis and gating strategy to detect CD56^{dim}CD16+ NK cells. Lower panel shows within the gate of CD56^{dim}CD16+ NK cells the percentage of CD107a-positive NK cells either without addition of any cells (neg. con.) or after addition of KIR3DL2-expressing HEK-293 cells without adding any Fc protein (-), or with addition of IgG1-Fc control protein, or with addition of KIR3DX1rep-lgG1-Fc protein. **B)** Scheme of the construct to express the extracellular part of FLAG-tagged KIR3DX1-repaired (leader, FLAG tag, D0, D1, D2 domains, stem) fused to the transmembrane and cytoplasmic part of CD28 and the cytoplasmic part of CD3zeta. This CAR-like KIR3DX1rep represents a stimulatory receptor. **C)** Expression of the CAR-KIR3DX1rep after transduction into NK-92 cells was measured with an anti-FLAG antibody. **D)** Left panel shows the CD107a expression of untransduced NK-92 after co-incubation with either KIR3DL2-expressing HEK-293 (uppermost curve), or HEK-293 expressing mutant KIR3DL2 (C319P-C279Y, second uppermost curve), or untransfected HEK-293 (third uppermost curve) or without co-incubation of any cells (neg. con., bottom curve). Right panel shows the CD107a expression of NK-92 transduced with with the construct shown in B) after co-incubation with either KIR3DL2-expressing HEK-293 (uppermost curve), or HEK-293 expressing mutant KIR3DL2 (C319P-C279Y, second uppermost curve), or untransfected HEK-293 (third uppermost curve), or without co-incubation of any cells (neg. con., bottom curve).

### Examples:

The human *KIR3DX1* gene comprises a 7 bp deletion in exon 5, which results in the expression of a truncated protein that does not form a functional KIR family receptor. The inventors thus sought to identify the reason for the functional inactivation of KIR3DX1 specifically in humans.

### Materials and Methods:

### Expression constructs used in this study:

IgG1-Fc
KIR3DX1rep-IgG1-Fc
KIR3DX1wt-lgG1-Fc
KIR3DL2-HA
KIR3DX1_LMP_TAP_KIR3DL2
D0-KIR3DX1-IgG1-Fc
D1-KIR3DX1-lgG1-Fc
D2-KIR3DX1 rep-lgG1-Fc
KIR3DX1 promoter containing KIR3DL2
FLAG-KIR3DX1rep
FLAG-KIR3DX1wt
D2-KIR3DL2-lgG1-Fc
D0-KIR3DL2-IgG1-Fc
KIR3DL2-C313P-mut
KIR3DL2-C279Y-mut
KIR3DL2-both C-mut
Lentiviral FLAG-KIR3DX1rep-CD28-CD3zeta

### Transfection, transduction and cells

Transfection of plasmid DNA construct into cells was performed using Lipofectamine 2000 following supplier's instructions (Thermo Fisher Scientific) or with Lonza's Kit-V for Nucleofector II electroporation-based transfection system according to the manufacturer's instructions or lentiviral particles were generated through a three-plasmid transfection system (lentiviral vector plasmid containing gene of interest, packaging plasmid containing genes encoding structural proteins (Gag, Pol, and Rev) and envelope plasmid containing VSV-G) and then cells were transduced with the generated particles. Cells were either analyzed 48-55 hr post-transfection (transient transfection) or stably transfected cells were obtained by selection with the plasmid DNA specific antibiotic for at least 14 days.

HEK-293, HUT78, Jurkat, YT and NK-92 cells were cultured in medium (DMEM + high Glucose + Stable Glutamine + 25 mM HEPES, 10% inactivated FBS and 0.1% Gentamicin), medium (IMDM + L-Glutamin + 25 mM HEPES, 10% inactivated FBS and 0.1% Gentamicin), medium (RPMI-1640 + L-Glutamin + 25 mM HEPES, 10% inactivated FBS and 0.1% Gentamicin), medium (RPMI-1640 + GlutaMAX, 20% inactivated FBS, 55 mm beta-mercaptoethanol and 0.1% Gentamicin), medium (Alpha MEM + Ribonucleosides + Deoxyribonucleosides, 12.5% Horse serum, 12.5% inactivated FBS, 2 mM L-Glutamine, 0.1% Gentamicin and 200 U/ml IL-2) at 37 °C with 5%, respectively.

Tube containing EDTA was used to collect human peripheral blood and the PBMCs layer was separated into leucosep tube with gradient centrifugation (800 x g for 40 min) using Pancoll human (density of 1.077g/ml, Pan Biotech). PBMCs were maintained in medium (RPMI-1640 + GlutaMax, 10% inactivated FBS, 0.1% Gentamicin, with or without 500 U/ml IL-2 and 10 ng/ml IL-15).

### Flow cytometry analysis

Detection of KIR3DL2 or mutated KIR3DL2 on the cell surface was performed by staining with either PE-conjugated mouse anti-human CD158e/k (KIR3DL1/DL2) antibody (clone 5.133, Miltenyi Biotec) or mouse anti-human KIR3DL2 monoclonal antibody (clone Q66; Pende et al. 1996 PMID: 8760804) and subsequent staining with a goat anti-mouse secondary antibody conjugated to PE. Recombinant human anti-FLAG IgG1 antibody conjugated to FITC (clone REA216, Miltenyi Biotec) was used to detect expression of FLAG-tagged KIR3DX1 at the cell surface. Staining of KIR3DL1, KIR3DS1, KIR2DL1, KIR2DL3 or KIR2DL5 expressing Jurkat cells was conducted using PE conjugated mouse anti-human CD158e/k (KIR3DL1/DL2, clone 5.133), FITC conjugated anti-human CD158e1/e2 (KIR3DL1/DS1) recombinant human IgG1 (clone REA168, Miltenyi Biotec), FITC conjugated mouse anti-human CD158a/h (KIR2DL1/DS1) antibody (clone 11PB6, Miltenyi Biotec), PerCP-conjugated mouse anti-human CD158b2 (KIR2DL3) antibody (clone 180701, R & D system) or APC conjugated mouse anti-human CD158f (KIR2DL5) antibody (clone UP-R1, Miltenyi Biotec).

To determine the binding of IgG1-Fc-tagged proteins, IgG1-Fc-tagged proteins were first added to the target cells and subsequently stained with APC-conjugated mouse anti-human IgG-Fc recombinant antibody (clone QA19A42, BioLegend).

To define NK cells in flow cytometry of PBMC samples, a cocktail of antibodies (collectively called lineage) against T cells, B cells, and monocytes was used in addition to NK cell marker CD56. NK cells were then defined as lineage-negative (-) and CD56-positive (+). For this, Brilliant Violet 650 conjugated mouse anti-human CD56 (clone 5.1H11, BioLegend) was applied in combination with mouse anti-human lineage cocktail 3 (lin 3, BD Bioscience) to exclude lineage markers (CD3, CD14, CD19 and CD20).

To measure degranulation of NK cells upon their stimulation with HEK-293 cells either with or without expressing KIR3DL2, HEK-293 target cells were incubated with IgG1-Fc-tagged KIR3DX1 proteins and co-incubated with PBMCs. Subsequently, CD107a expression was determined on the cell surface of NK cells by flow cytometry. NK cells in the PBMCs were defined as lin-CD16+CD56+ cells (staining of CD16 using PerCP/Cyanine5.5 conjugated mouse anti-human CD16 antibody, clone 3G8, BioLegend) to measure the percentage of CD107a (mouse anti-human CD107a conjugated with PE, clone H4A3, BD Biosciences)-positive NK cells upon stimulation.

Degranulation of NK-92 or NK-92 cells expressing FLAG-KIR3DX1rep-CD28-CD3zeta after stimulation with KIR3DL2-expressing HEK-293 cells was determined by measuring CD107a+ NK-92 cells.

### Co-Immunoprecipitation (Co-IP) and Western blot analysis

HEK-293 cells stably expressing HA-tagged KIR3DL2 were lysed in ice cold NP-40 (Fluka) lysis buffer. The lysate sample was pre-cleared using Protein G sepharose beads (GE healthcare) and the protein concentration was determined in a Qubit 4 fluorometer using Qubit protein assay kits (Thermo Fisher Scientific). Subsequently, IgG1-Fc or IgG1-Fc-tagged KIR3DX1 proteins were added to the pre-cleared lysate and incubated overnight at 4°C with rotation. Next day, protein G beads were added to incubate another 5 hours at 4°C. After washing of the beads, bound proteins were eluted in 5x SDS-PAGE reducing protein loading buffer (Bosterbio) after incubation at 95°C for 5 minutes. Lysate or co-immunoprecipitated protein samples were resolved in 8-16% Mini protean-TGX stain free protein gels (Bio-Rad) and blotted onto a Trans-Blot Turbo mini PVDF (Bio-Rad) using the Trans-Blot Turbo Transfer-System (Bio-Rad). A mouse anti-HA antibody (clone HA-7, Sigma-Aldrich) followed by a polyclonal goat anti-mouse antibody conjugated to horseradish peroxidase secondary antibody (Merckmillipore) diluted with Everyblot blocking buffer (Bio- Rad) and Immobilon Forte Western HRP Substrate (Merck Millipore) solution was applied to detect chemiluminescent signal of HA-tagged proteins. ECL Chemocam Imager (Intas) equipped with ChemoStar imager software for Windows was used to capture the image of the blot.

### Figure preparation

FlowJo version 10.8.0, GraphPad Prism 9 statistical analysis software and PowerPoint for version 16.75 for MAC OS were used for the preparation, analysis, arrangement and annotation of the figures.

### Results:

### Identification of KIR3DL2 as binding partner of KIR3DX1

Screening of available primate genome sequences indicated that humans are the only primate species where *KIR3DX1* carries a deletion in its open-reading frame. This 7 bp deletion in exon 5 is also detectable in the available Neanderthal and Denisova genomes (data not shown), indicating that introduction of the deletion and its homozygous fixation occurred in the lineage leading to *Homo sapiens* after separation from chimpanzees. Comparison of primate sequences revealed that the deleted sequence is conserved in nonhuman primates (Figure 1). In order to produce a human KIR3DX1 in its ancient form, we introduced the conserved 7 bp into the human *KIR3DX1* cDNA (now designated KIR3DX1-repaired or KIR3DX1 rep). The extracellular part of KIR3DX1 rep was then fused to the Fc region of human IgG1 and expressed in HEK-293 cells to produce a soluble KIR3DX1rep-Fc protein, which was used to stain human PBMCs in flow cytometry. About 18% of PBMCs stained positive with KIR3DX1rep-Fc (Figure 2A). In order to identify the binding protein, we subjected KIR3DX1rep-Fc to pull-down analysis of human PBMCs and subsequent mass spectrometry analysis. Surprisingly, KIR3DL2 was among the list of identified proteins from this pull-down.

The specific binding of KIR3DX1rep-Fc to KIR3DL2 was verified using KIR3DL2-transfected HEK-293 cells as well as human cutaneous T cell lymphoma cell line HUT78, which is known to endogenously express KIR3DL2. Binding of KIR3DX1rep to KIR3DL2 was verified by pull-down assay using a C-terminally HA-tagged KIR3DL2 expressed in HEK-293 cells and KIR3DX1rep-Fc (Figure 2B) as well as in flow cytometry analysis, where binding of KIR3DX1rep-Fc to KIR3DL2-expressing HEK-293 was detectable but not binding of the wildtype KIR3DX1 (Figure 2C). Usage of both anti-human IgG (to detect KIR3DX1rep-Fc) and anti-KIR3DL2 monoclonal antibody Q66 (or anti-human KIR3DL1/KIR3DL2 monoclonal antibody) did not result in detectable simultaneous binding, most probably because binding of the two proteins masked the epitopes recognized by these monoclonal antibodies (Figure 2D). Thus, we expressed a FLAG-tagged version of KIR3DX1rep-Fc as well as a V5-tagged version of KIR3DL2 and were able to detect KIR3DX1rep-Fc via the anti-FLAG antibody only on those cells that also expressed KIR3DL2. Figure 2E shows expression of KIR3DL2 on HUT78 Sézary syndrome cells and binding of KIR3DX1rep-Fc to HUT78. The wildtype KIR3DX1-Fc protein does not detectably bind to HUT78 cells (Figure 2E).

We also tested whether KIR3DX1rep can bind other human KIR proteins and used Jurkat cells that were transfected to express single human KIR proteins. As shown in Figure 3, low level of binding was detectable for KIR3DL1, but not for other KIRs including KIR3DS1.

### Repaired KIR3DX1 binds KIR3DL2 via its D2 domain

We were then interested in identifying the KIR3DX1rep domain that is involved in binding KIR3DL2. For this, we produced single D0, D1 and D2(rep) domains as Fc fusion proteins. As shown in Figure 4A, only the repaired D2 domain specifically bound to KIR3DL2-expressing cells. We also expressed full-length KIR3DX1 in its repaired form as cell surface receptor and containing a FLAG tag for detection with an anti-FLAG antibody on transfected cells (Figure 4B) and tested which domain of KIR3DL2 is involved in binding. The D0 and D2 but not the D1 domain of KIR3DL2 were produced as Fc fusion protein, and it was found that that the D0 domain specifically bound KIR3DX1rep (Figure 4C). As low binding of KIR3DX1rep was observed for KIR3DL1 but no binding for KIR3DS1, we compared the D0 sequences of KIR3DL1, KIR3DS1 and KIR3DL2. As shown in Figure 4D, the glycine residue at position 56 is the only amino acid residue that differentiates the non-binder KIR3DS1 from the binder KIR3DL1 and KIR3DL2. As shown in figure 4E, mutation of amino acid position 56 from serine into glycine did not change cell surface expression level of KIR3DL2, but abrogated binding of KIR3DX1rep. Thus, specific binding of KIR3DL2 and KIR3DX1rep involves the D0 domain of KIR3DL2 at position 56 and most probably the part of the D2 domain of KIR3DX1 that was affected by the 7 bp deletion as the wildtype KIR3DX1 protein is not able to bind KIR3DL2.

### KIR3DX1rep binds KIR3DL2 only in its homodimeric form

Two cysteine residues at position 279 and 313 distinguish KIR3DL2 from the other human and nonhuman primate KIR proteins. These cysteines are thought to be responsible for the known homodimerization of this receptor. We mutated either one (C279Y or C313P) or both cysteine residues to amino acid residues that were invariably found in other KIR proteins at these two positions and expressed these three variants in HEK-293 cells. Successful expression of the individual KIR3DL2 mutants could be shown (Figure 4F). Neither the two single cysteine mutants nor the double mutant KIR3DL2 appeared to be bound by KIR3DX1rep -Fc (Figure 4F). Thus, we conclude from these experiments that the repaired KIR3DX1 can only bind to KIR3DL2 when both KIR3DL2-specific cysteines at positions 279 and 313 are present, and therefore, KIR3DX1rep most probably binds only the homodimerized form of KIR3DL2.

### Repaired KIR3DX1 can be used to detect and destroy KIR3DL2-expressing cells

KIR3DL2 is a marker on cutaneous T cell lymphoma (Bagot et al., 2001; Sicard et al., 2015; Schmitt et al., 2017; Roelens et al., 2022) such as Sézary syndrome. In order to figure out whether the repaired KIR3DX1 can be exploited in immunotherapy against KIR3DL2-expressing cancer cells, we established a stimulatory receptor by fusing the extracellular part of FLAG-tagged KI R3DX 1 rep with the transmembrane and cytoplasmic region of human CD28 and CD3zeta (Figure 5B). This stimulatory KIR3DX1rep was stably transfected in the NK92 NK cell line. Expression on the cell surface of these cells was verified by using an anti-FLAG tag antibody (Figure 5C). Indeed, stimulatory KIR3DX1rep-expressing NK92 cells showed increased degranulation as compared to untransfected parental NK92 cells upon co-incubation with HEK-293 cells expressing KIR3DL2, but no noticeable increase in degranulation was detected after co-incubation with HEK-293 cells expressing the cysteine mutants of KIR3DL2 (Figure 5D). Thus, KIR3DX1rep can bind KIR3DL2 not only in the soluble Fc-tagged form, but also in membrane-bound form as a receptor on the cell surface of killer cells.

Besides cellular immunotherapy, also the usage of a soluble KIR3DX1rep-Fc protein is an option for immune therapy to target KIR3DL2-expressing cancer cells. To test whether soluble KIR3DX1rep-Fc protein bound to KIR3DL2-expressing cells induced antibody-dependent cellular cytotoxicity (ADCC), we incubated KIR3DL2+ HEK-293 cells with KIR3DX1-Fc and then co-incubated these cells with human PBMCs. As shown in Figure 5A, the incubation with KIR3DX1rep-Fc but not the KIR3DX1wt-Fc significantly enhanced degranulation (CD107a assay) on CD16+ NK cells. Thus, the repaired KIR3DX1 protein offers two principal opportunities for immune therapy against KIR3DL2-expressing cells: usage of a soluble protein and of a cell-based stimulatory receptor as therapeutic compound.

### Repaired KIR3DX1 can be used to interfere with Th17 cell differentiation and survival

KIR3DL2 is not only a marker on cutaneous T cell lymphoma but plays also an important role in autoimmune diseases involving the HLA-B*27 allotype (Chan et al., 2005; Bowness et all, 2011; Cauli et al., 2013; Hurabielle et al., 2017, Hurabielle et al., 2018; Khan et al., 2023). Ankylosing spondylitis (AS) or Morbus Bechterew is an autoimmune disease that has a strong genetic linkage with the HLA-B*27:05 allele. The molecular mechanism of disease development is thought to involve two steps in HLA-B*27-positive patients: the first step is the induced expression of KIR3DL2 on activated CD4+ Th cells, and the second step is the molecular interaction of KIR3DL2 with HLA-B*27, which licenses CD4+ Th cells to develop into inflammatory Th17 cells expressing increased amounts of IL-17 and reduced amounts of IL-2 (Chan et al., 2005; Bowness et al., 2011; Ridley et al., 2016; Wong-Baeza et al., 2013). Therefore, blocking interaction of KIR3DL2 with HLA-B*27 through soluble KIR3DX1rep in a form that does not lead to ADCC may help in therapy of such diseases, in particular, it may prevent an increase in severity of the disease.

To study this interaction, we produced 721.221 cells expressing HLA-B*27:05. The human 721.221 B lymphoma cell line lacks HLA-A, HLA-B, and HLA-C expression and, therefore, is particularly well suited to express transfected HLA class I genes for subsequent functional studies.

CD4+ T cells are isolated from healthy donors and from AS patients and stimulated with CD2/CD3/CD28 beads to induce KIR3DL2 expression. During this stimulation, the cells are co-incubated with 721.221-HLA-B*27:05 cells to further increase KIR3DL2 expression (Ridley et al., 2016). According to Ridley and co-workers (Ridley et al., 2016) this leads to differentiation into Th17 that increase expression of the pro-inflammatory IL-17 cytokine and decrease expression of IL-2. Soluble KIR3DX1rep protein (after removal of the Fc portion, or by using a Fc derived from human IgG4 that does not induce ADCC) in this assay is used to test whether the presence of KIR3DX1rep and the expected binding to KIR3DL2 interferes with interaction of KIR3DL2 with HLA-B*27 and, thereby, inhibits the differentiation into Th17 cells and lowers the expression of the pro-inflammatory IL-17 cytokine. Thus, soluble KIR3DX1rep may be used to treat patients with autoimmune diseases in which the interaction of KIR3DL2 with HLA-B*27 plays a role, such as AS.

### Repaired KIR3DX1 can be used to interfere with binding of CpG oligos to KIR3DL2 and subsequent activation of KIR3DL2-expressing cells

Pathogen-associated molecular patterns (PAMPs) are strong inductors of immune responses as they clearly distinguish self from non-self, i.e. pathogens. Such PAMPs also include DNA containing an unmethylated cytosine followed by a guanine, which is characteristic of bacterial but not for mammalian DNA. CpG oligos mimic such bacterial DNA and, thus, bacterial infection. The cellular receptor for CpG DNA (and oligos) is toll-like receptor 9 (TLR9), which upon binding of the CpG oligo stimulates the expression of pro-inflammatory cytokines such as IFN-gamma and increase activity of cytotoxic cells (Sivori et al., 2010a; Sivori et al., 2010b; Sivori et al., 2004). TLR9 is located in the endoplasmic reticulum and is translocated to the endosomal compartment upon exposure of the cell with CpG (Latz et al., 2004). It was shown by Sivori et al., 2010a that KIR3DL2 is a receptor for CpG oligos, which binds this PAMP via its D0 domain. The KIR3DL2 is endocytosed upon binding and shuttles the CpG to the endosomal compartment where the CpG cargo is loaded on TLR9. This process leads to expression of interferon-gamma and to elevated cytotoxicity in NK cells.

As the D2 domain of KIR3DX1rep binds the D0 domain of KIR3DL2, we hypothesized that this binding reduces or abrogates CpG oligo binding of KIR3DL2. We thus established cells that constitutively express KIR3DL2 and in addition harbor KIR3DX1rep that can be induced by the addition of tetracycline (tet-on expression system). This approach offers the advantage that we can analyze the same cells under normal condition (KIR3DX1rep protein not present) and under induced condition (KIR3DX1rep protein present).

Firstly, we test whether the addition of CpG induces the expected KIR3DL2 internalization in normal condition and compare these results with the internalization of KIR3DL2 under induced conditions. Secondly, we measure the functional consequences of this experimental set-up by measuring expression of IFN-gamma. The three KIR3DL2 cysteine mutants are also tested to see whether they can also efficiently internalize KIR3DL2 upon CpG oligo binding.

These experimental set-ups show whether homodimerization of KIR3DL2 is important for CpG oligo transport, and why KIR3DX1 was inactivated by the 7 bp deletion in the D2 domain-encoding exon 5, possibly, as a bound KIR3DX1 might interfere with the important function to sense and transport CpG DNA to TLRs.

Soluble KIR3DX1rep (either without IgG1-Fc or by using IgG4-Fc) is used to study whether the binding of the soluble KIR3DX1rep interferes with KIR3DL2's ability to bind CpG oligos. If this is the case, this opens up opportunities to use soluble KIR3DX1rep for treating pathogenic immune responses such as sepsis associated with severe bacterial infections.

Thus, soluble KIR3DX1 may be used to treat overshooting and finally pathogenic immune responses provoked by bacterial infections.

### References:

Sivori S, Falco M, Carlomagno S, Romeo E, Soldani C, Bensussan A, Viola A, Moretta L, Moretta A. A novel KIR-associated function: evidence that CpG DNA uptake and shuttling to early endosomes is mediated by KIR3DL2. Blood (2010a) 116:1637-1647. doi: 10.1182/blood-2009-12-256586.
   https://classic.clinicaltrials.gov/ct2/show/NCT02593045
   https://classic.clinicaltrials.gov/ct2/show/NCT03322618
Guethlein LA, Abi-Rached L, Hammond JA, Parham P. The expanded cattle KIR genes are orthologous to the conserved single-copy KIR3DX1 gene of primates. Immunogenetics (2007) 59:517-522. doi: 10.1007/s00251-007-0214-x.
Guethlein LA, Norman PJ, Hilton HHG, Parham P. Co-evolution of MHC class I and variable NK cell receptors in placental mammals. Immunological reviews (2015) 267:259-282. doi: 10.1111/imr. 12326.
Sambrook JG, Bashirova A, Andersen H, Piatak M, Vernikos GS, Coggill P, Lifson JD, Carrington M, Beck S. Identification of the ancestral killer immunoglobulin-like receptor gene in primates. BMC Genomics (2006) 7:209. doi: 10.1186/1471-2164-7-209
Yu, J., Song, Y. & Tian, W. How to select IgG subclasses in developing anti-tumor therapeutic antibodies. J Hematol Oncol 13, 45 (2020). https://doi.org/10.1186/s13045-020-00876-4.
Bowness P, Ridley A, Shaw J, Chan AT, Wong-Baeza I, Fleming M, Cummings F, McMichael A, Kollnberger S. Th17 cells expressing KIR3DL2+ and responsive to HLA-B27 homodimers are increased in ankylosing spondylitis. J Immunol. 2011 Feb 15;186(4):2672-80. doi: 10.4049/jimmunol.1002653. Epub 2011 Jan 19. PMID: 21248258; PMCID: PMC3210561.
Chan AT, Kollnberger SD, Wedderburn LR, Bowness P. Expansion and enhanced survival of natural killer cells expressing the killer immunoglobulin-like receptor KIR3DL2 in spondylarthritis. Arthritis and rheumatism (2005) 52:3586-3595. doi: 10.1002/art.21395.
Ridley A, Hatano H, Wong-Baeza I, Shaw J, Matthews KK, Mossawi HA, Ladell K, Price DA, Bowness P, Kollnberger S. Activation-Induced Killer Cell Immunoglobulin-like Receptor 3DL2 Binding to HLA-B27 Licenses Pathogenic T Cell Differentiation in Spondyloarthritis. Arthritis & rheumatology (Hoboken, NJ) (2016) 68:901-914. doi: 10.1002/art.39515.
Wong-Baeza I, Ridley A, Shaw J, Hatano H, Rysnik O, McHugh K, Piper C, Brackenbridge S, Fernandes R, Chan A, et al. KIR3DL2 Binds to HLA-B27 Dimers and Free H Chains More Strongly than Other HLA Class I and Promotes the Expansion of T Cells in Ankylosing Spondylitis. Journal of immunology (Baltimore, Md : 1950) (2013) 190:3216-3224. doi: 10.4049/jimmunol. 1202926.
Bagot M, Moretta A, Sivori S, Biassoni R, Cantoni C, Bottino C, Boumsell L, Bensussan A. CD4(+) cutaneous T-cell lymphoma cells express the p140-killer cell immunoglobulin-like receptor. Blood (2001) 97:1388-1391. doi: 10.1182/blood.v97.5.1388
Sicard H, Bonnafous C, Morel A, Bagot M, Bensussan A, Marie-Cardine A. A novel targeted immunotherapy for CTCL is on its way: Anti-KIR3DL2 mAb IPH4102 is potent and safe in non-clinical studies. Oncoimmunology (2015) 4:e1022306. doi: 10.1080/2162402x.2015.1022306
Schmitt C, Marie-Cardine A, Bensussan A. Therapeutic Antibodies to KIR3DL2 and Other Target Antigens on Cutaneous T-Cell Lymphomas. Frontiers in immunology (2017) 8:1010. doi: 10.3389/fimmu.2017.01010
Roelens M, Masson A, Andrillon A, Ram-Wolff C, Biard L, Boisson M, Mourah S, Battistella M, Toubert A, Bagot M, et al. Mogamulizumab induces long term immune restoration and reshapes tumor heterogeneity in Sézary syndrome. Brit J Dermatol (2022) doi: 10.1111/bjd.21018
Cauli A, Shaw J, Giles J, Hatano H, Rysnik O, Payeli S, McHugh K, Dessole G, Porru G, Desogus E, et al. The arthritis-associated HLA-B*27:05 allele forms more cell surface B27 dimer and free heavy chain ligands for KIR3DL2 than HLA-B*27:09. Rheumatology (Oxford, England) (2013) 52:1952-1962. doi: 10.1093/rheumatology/ket219
Hurabielle C, Thonnart N, Ram-Wolff C, Sicard H, Bensussan A, Bagot M, Marie-Cardine A. Usefulness of KIR3DL2 to Diagnose, Follow-Up, and Manage the Treatment of Patients with Sézary Syndrome. Clinical cancer research: an official journal of the American Association for Cancer Research (2017) 23:3619-3627. doi: 10.1158/1078-0432.ccr-16-3185
Hurabielle C, Leboeuf C, Ram-Wolff C, Meignin V, Rivet J, Vignon-Pennamen MD, Bonnafous C, Sicard H, Fite C, Raffoux E, et al. KIR3DL2 expression in patients with adult T-cell lymphoma/leukaemia. The British journal of dermatology (2018) 179:197-199. doi: 10.1111/bjd. 16322
Khan MA, Yong S, Wei JC. Ankylosing spondylitis: History, epidemiology, and HLA-B27. Int J Rheum Dis (2023) 26:413-414. doi: 10.1111/1756-185x.14547
Sivori S, Falco M, Moretta L, Moretta A. Extending killer Ig-like receptor function: from HLA class I recognition to sensors of microbial products. Trends in immunology (2010b) 31:289-294.
Sivori S, Falco M, Chiesa MD, Carlomagno S, Vitale M, Moretta L, Moretta A. CpG and double-stranded RNA trigger human NK cells by Toll-like receptors: induction of cytokine release and cytotoxicity against tumors and dendritic cells. Proceedings of the National Academy of Sciences of the United States of America (2004) 101:10116-10121. doi: 10.1073/pnas.0403744101
Latz E, Schoenemeyer A, Visintin A, Fitzgerald KA, Monks BG, Knetter CF, Lien E, Nilsen NJ, Espevik T, Golenbock DT. TLR9 signals after translocating from the ER to CpG DNA in the lysosome. Nature immunology (2004) 5:190-198. doi: 10.1038/ni1028
Felices M, Lenvik TR, Davis ZB, Miller JS, Vallera DA. Generation of BiKEs and TriKEs to Improve NK Cell-Mediated Targeting of Tumor Cells. Methods Mol Biol. 2016;1441:333-46. doi: 10.1007/978-1-4939-3684-7_28. PMID: 27177679; PMCID: PMC5823010.

## Claims

1. A polypeptide capable of binding to human KIR3DL2 and comprising an amino acid sequence having at least 85% sequence identity to SEQ ID NO: 1, wherein the polypeptide does not comprise an amino acid sequence of SEQ ID NO: 15, 16, 17, 36 or 60, and wherein, preferably, the polypeptide comprises the amino acid sequence WSX₁X₂S, wherein X₁ is any natural amino acid and X₂ is S or P.

2. The polypeptide of claim 1 comprising SEQ ID NO: 2, optionally, comprising SEQ ID NO: 3.

3. The polypeptide of any of claims 1 or 2, wherein the polypeptide further comprises a D0 domain of KIR3DX1 consisting of an amino acid sequence having at least 70% sequence identity to SEQ ID NO: 5.

4. The polypeptide of any of claims 1-3, wherein the polypeptide further comprises a D1 domain of KIR3DX1 consisting of an amino acid sequence having at least 70% sequence identity to SEQ ID NO: 6, wherein, preferably, the polypeptide comprises both a D0 domain and a D1 domain of KIR3DX1 wherein, optionally, the polypeptide comprises an amino acid sequence of SEQ ID NO: 7.

5. A polypeptide of any of any of claims 1-4, wherein the polypeptide further comprises a stem region, a transmembrane region and a cytoplasmic region of a killer cell immunoglobulin-like receptor (KIR), wherein, optionally, the polypeptide comprises a stem region comprising an amino acid sequence having at least 70% sequence identity to SEQ ID NO: 10, a transmembrane region comprising an amino acid sequence having at least 70% sequence identity to SEQ ID NO: 11 and a cytoplasmic region comprising an amino acid sequence having at least 70% sequence identity to SEQ ID NO: 12.

6. A fusion protein comprising
a) a polypeptide of any of claims 1-4 or a polypeptide comprising an amino acid sequence of any of SEQ ID NO: 15, 16, 17, 36 or 60 fused to an Fc region of an immunoglobulin, wherein, preferably, the Fc region is an IgG1, an IgG2, IgG3 or IgG4 Fc region.
b) a polypeptide of any of claims 1-4 or a polypeptide comprising an amino acid sequence of any of SEQ ID NO: 15, 16, 17, 36 or 60 linked to at least one additional antigen-binding moiety.

7. A chimeric antigen receptor (CAR) comprising a polypeptide of any of any of claims 1-4 or a polypeptide comprising an amino acid sequence of any of SEQ ID NO: 15, 16, 17, 36 or 60 as an antigen recognition domain, wherein the CAR further comprises a transmembrane domain, an intracellular signaling domain, an intracellular co-stimulatory domain and, optionally, a hinge domain located between the antigen recognition domain and the transmembrane domain.

8. The CAR of claim 7, wherein the CAR has an amino acid sequence of SEQ ID NO: 14 or at least 90% sequence identity thereto.

9. A nucleic acid encoding the polypeptide of any of claims 1-5, the fusion protein of claim 6 or the CAR of any of claims 7 or 8.

10. A cell comprising a nucleic acid of claim 9, and preferably expressing a polypeptide of claim 5 or a CAR of any of claims 7 or 8, or a cell expressing a polypeptide corresponding to SEQ ID NO: 33, 34, 35, 38 or 62,
wherein, optionally, the cell is a lymphoid cell selected from the group comprising a natural killer cell, a CD8⁺ cytotoxic T cell, a natural killer T cell or a precursor thereof.

11. A pharmaceutical composition comprising
(a) a polypeptide of any of claims 1-5;
(b) a polypeptide comprising an amino acid sequence of any of SEQ ID NO: 15, 16, 17, 36 or 60;
(c) a fusion protein of claim 6;
(d) a nucleic acid of claim 9; and/or
(e) a cell of claim 10.

12. The pharmaceutical composition of claim 11 for use in a method of treating a rheumatic disease in a subject, wherein the rheumatic disease preferably is a spondyloarthritis (SpA) such as ankylosing spondylitis (AS).

13. The pharmaceutical composition of claim 11 for use in treating or preventing a condition associated with a TLR9-mediated inflammatory response, wherein optionally, the condition is sepsis or bacterial infection, such as a bacterial superinfection.

14. The pharmaceutical composition of claim 11 for use in a method of treating of a cancer expressing KIR3DL2, wherein the cancer expressing KIR3DL2 is selected from the group comprising cutaneous T-cell lymphoma (CTCL), Sézary syndrome, mycosis fungoides, CD30⁺ cutaneous lymphoma, adult T-cell leukemia (ATL) or myelodysplastic syndrome.

15. An in-vitro method for detecting KIR3DL2 expression in a subject, comprising contacting a sample obtained from a subject with a polypeptide of any of claims 1 to 5, a fusion protein of claim 6 or a polypeptide comprising an amino acid sequence of any of SEQ ID NO: 15, 16, 17, 36 or 60, wherein the polypeptide or the fusion protein optionally is linked to a label.
